# EUROPEAN PATENT SPECIFICATION

(11) **EP 3 278 811 B1**
(45) Date of publication and mention of the grant of the patent: **02.12.2020**
(21) Application number: 17187879.6
(22) Date of filing: 29.03.2013
(51) Int. Cl.: A61K 38/18, A61P 25/02, A61P 43/00, A61P 15/10

(54) **USE OF GGF2 TO TREAT NEUROPATHIC PAIN UPON PERIPHERAL NERVE INJURY**
VERWENDUNG VON GGF2 ZUR BEHANDLUNG VON NEUROPATHISCHEN SCHMERZEN DURCH PERIPHERE NERVENLÄSIONEN
UTILISATION DE GGF2 POUR TRAITER UNE DOULEUR NEUROPATHIQUE DUE À UNE LÉSION DU NERF PÉRIPHÉRIQUE

(30) Priority: 30.03.2012 US 201261618381 P; 20.07.2012 US 201261674060 P; 27.08.2012 US 201261693589 P; 27.08.2012 US 201261693585 P; 14.03.2013 US 201361785419 P
(43) Date of publication of application: 07.02.2018
(62) Divisional of application: 13716919.9
(73) Proprietor: Acorda Therapeutics, Inc., Ardsley, NY 10502 (US)
(72) Inventor: CAGGIANO, Anthony, O., Larchmont, NY New York 10538 (US); BELLA, Anthony, J., Ottawa, Ontario K2R 1AB (CA); GANGULY, Anindita, White Plains, NY New York 10605 (US); IACI, Jennifer, Boonton, NJ New Jersey 07005 (US); PARRY, Thomas, Hellertown, PA Pennsylvania 18055 (US); COLBURN, Raymond, Warren, Chalfont, PA Pennsylvania 18914 (US)
(74) Representative: Haseltine Lake Kempner LLP

(56) References cited:
- WO-A1-96/15812
- WO-A1-99/18976
- WO-A2-2009/108390
- WO-A2-2011/047183
- WO-A2-2012/021818

## Description

### FIELD OF THE DISCLOSURE

This invention relates generally to the fields of molecular biology, regenerative medicine, and nerve trauma or injury. Methods of the disclosure are used to administer neuregulin isoforms or functional segments thereof to prevent, treat, or ameliorate symptoms of peripheral nerve injury. Subjects of these methods either a risk of incurring a peripheral nerve injury or have a peripheral nerve injury.

### BACKGROUND

Peripheral nerves are commonly injured from trauma including automobile accidents, motorcycle accidents, surgeries, knife and projectile wounds, and birth injuries to both the child and mother. Following nerve injury there is a loss of sensation and/or function in the regions of the body innervated by the damaged nerve. Common surgical causes of nerve injury include prostatectomy and mastectomy. For example, following nerve injury from prostatectomy there is commonly erectile dysfunction.

There is a need for additional therapies and treatments for peripheral nerve injuries. Moreover, there is a long-felt need in the art for a therapy that can both prevent and/or limit the extent of dysfunction following a nerve injury.

WO9918976 discloses methods for treatment and/or prophylaxis of certain neurological-related disorders. WO2011047183 discloses the use of neuregulins to prevent or treat peripheral nerve injury, to attenuate, ameliorate or avoid the loss of peripheral nerve function. WO9615812 discloses methods of affecting cellular communication in a vertebrate. WO2009108390 discloses the administration of glial growth factor 2 to a patient in need thereof, to achieve serum levels of glial growth factor 2 within a desired therapeutic window. WO2012021818 discloses methods of treating central nervous injury using glial growth factor 2.

### SUMMARY

Neuregulins have been implicated as neuroprotective and neurorestorative effects in a variety of animal models of central nervous system diseases and injuries. Herein disclosed are methods of treating or ameliorating peripheral nerve injury by administering a neuregulin or a functional segment thereof to a subject that has a peripheral nerve injury or is at risk of peripheral nerve injury. Further disclosed are methods of treating or ameliorating peripheral nerve injury by administering a GGF2 or a functional segment thereof to the subject.

The present disclosure demonstrates that neuregulin treatment of peripheral nerve injury can attenuate the loss of peripheral nerve function, ameliorate or attenuate loss of peripheral nerve function when given either before or after nerve injury, and in some instance restore peripheral nerve function.

Accordingly, the present invention provides a composition comprising an effective amount of a GGF2 for use in a method to decrease neuropathic pain in a subject at risk of incurring a peripheral nerve injury due to a surgical procedure

The term subject includes mammals, and particularly human subjects.

Peripheral nerve injury (PNI) in humans may be acquired through physical trauma or may be a result of disease. For example, peripheral nerves are injured from trauma including automobile accidents, motorcycle accidents, surgeries, knife and projectile wounds, and birth injuries to both the child and mother. Complications following injury may lead to deficits in sensorimotor function and autonomic dysregulation.

Although all peripheral nerves are contemplated, the disclosed methods may be applied to the following non-limiting examples of peripheral nerves, including the sciatic and cavernous nerves.

Injury to the sciatic nerve results in a loss of muscle mass, weakness of the muscle, target muscle atrophic changes, paresis, and pain, often reducing the capacity for rehabilitation an impacting quality of life. For example, with respect to injury to the sciatic nerve, the compositions of the disclosure increase muscle mass, improve muscle strength, reduce muscular atrophy, and reduce neuropathic pain and enhance sensory recovery. Results from non-invasive 3-dimensional (3D) ultrasound imaging have demonstrated significant reductions in both gastrocnemius and soleus muscle volumes following crush and transection on the injured hind limb over time compared to sham animals. The administration of GGF2, for example, increases myofiber size and reduces fibrosis in a tissue surrounding the peripheral nerve. Moreover, the administration of GGF2, for example increases myofiber size and reduces fibrosis in skeletal muscle.

A peripheral nerve injury may result from a surgery, such as a prostatectomy. In the context of essentially any surgical intervention, peripheral nerve injury may be the direct result of tissue dissection, tissue resection and/or secondary to limb positioning and/or compression.

The rat erectile dysfunction model is used as an in vivo system to demonstrate the effectiveness of neuregulins in treating peripheral nerve injury. Neuregulin can be effective as a monotherapy for any peripheral nerve injury and does not require co-treatment with natural or artificial nerve conduits or co-treatment with cellular therapies such as Schwann cells. A neuregulin-1 peptide (GGF2) was tested in a bilateral crush model in rat, which is an accepted model of cavernous nerve injury. This model has been used to test sildenifil and other ED drugs. As shown in the examples provided, GGF2 improved functional outcomes when nerves were electrostimulated 5 weeks following injury and intercavernosal pressure (ICP) was measured.

Exemplary surgical procedures include, but are not limited to, making an incision in the subject and performing a caesarian section. In an aspect of this embodiment, exemplary types of the peripheral nerve injury include, but are not limited to, a nerve transection, a nerve crush, and a nerve demyelination. In an aspect of this embodiment, the peripheral nerve may be a sciatic nerve. Alternatively, or in addition, the peripheral nerve may be a cavernous nerve.

Exemplary surgical procedures include, but are not limited to, tissue dissection or tumor resection surgery. Alternatively, or in addition, exemplary surgical procedures include, but are not limited to, a pelvic, abdominal, or colorectal surgery. In an aspect of this embodiment, a tissue or tumor dissected or removed during surgery may malignant or benign. Malignant tissue or tumors may be one or more types of cancer. Exemplary types of cancer include, but are not limited to solid cancers, prostate cancer, and breast cancer. In an aspect of this embodiment, exemplary types of the peripheral nerve injury include, but are not limited to, a nerve transection, a nerve crush, and a nerve demyelination. In an aspect of this embodiment, the peripheral nerve may be a sciatic nerve. Alternatively, or in addition, the peripheral nerve may be a cavernous nerve. In an aspect of this embodiment, the peripheral nerve injury may result in erectile dysfunction.

In an embodiment of the invention, a peripheral nerve injury may be due to delivery of an infant. Exemplary surgical procedures include, but are not limited to, making an incision in the subject and performing a caesarian section. In an aspect of this embodiment, exemplary types of the peripheral nerve injury include, but are not limited to, a nerve transection, a nerve crush, and a nerve demyelination. In an aspect of this embodiment, the peripheral nerve may be a sciatic nerve. Alternatively, or in addition, the peripheral nerve may be a cavernous nerve.

In an embodiment of the invention, the composition fis suitable for administration prior to incurring the peripheral nerve injury. In an aspect of this embodiment, the composition may be administered prior to but not after incurring the peripheral nerve injury. The composition may be administered according to a discontinuous dosing regimen. Preferably, the discontinuous dosing regimen comprises administering the composition at specified intervals. Exemplary specified intervals may include, but are not limited to, administration at intervals of every 24 hours, every 48 hours, and every 72 hours. Alternatively, specified intervals may include, but are not limited to, administration at intervals of at least every 24 hours, at least every 48 hours, and at least every 72 hours. In an aspect of this embodiment, the discontinuous dosing regimen may be continued for a period of time selected from the group consisting of about 1 week, about 2 weeks, about 3 weeks, about 4 weeks, about 5 weeks, about 6 weeks, about 7 weeks, about 8 weeks, about 9 weeks, about 10 weeks, about 11 weeks, and about 12 weeks. In an aspect of this embodiment, the specified interval of administration may be at least once per week. In an aspect of this embodiment, the specified interval of administration may be at least once per week and the discontinuous dosing regimen may continue for a period of time selected from the group consisting of about 1 month, about 2 months, and about 3 months.

In an embodiment of the invention, the composition is suitable for administration in the course of incurring the peripheral nerve injury. In an aspect of this embodiment, the composition may be administered in the course of but not after incurring the peripheral nerve injury. A neuregulin or a composition comprising a neuregulin may be administered according to a discontinuous dosing regimen. Preferably, the discontinuous dosing regimen comprises administering the composition at specified intervals. Exemplary specified intervals may include, but are not limited to, administration at intervals of every 24 hours, every 48 hours, and every 72 hours. Alternatively, specified intervals may include, but are not limited to, administration at intervals of at least every 24 hours, at least every 48 hours, and at least every 72 hours. In an aspect of this embodiment, the discontinuous dosing regimen may be continued for a period of time selected from the group consisting of about 1 week, about 2 weeks, about 3 weeks, about 4 weeks, about 5 weeks, about 6 weeks, about 7 weeks, about 8 weeks, about 9 weeks, about 10 weeks, about 11 weeks, and about 12 weeks. In an aspect of this embodiment, the specified interval of administration may be at least once per week. In an aspect of this embodiment, the specified interval of administration may be at least once per week and the discontinuous dosing regimen may continue for a period of time selected from the group consisting of about 1 month, about 2 months, and about 3 months.

In an embodiment of the invention, the composition is suitable for administration prior to and in the course of incurring the peripheral nerve injury. In an aspect of this embodiment, the composition may be administered prior to and in the course of but not after incurring the peripheral nerve injury. The composition may be administered according to a discontinuous dosing regimen. Preferably, the discontinuous dosing regimen comprises administering the composition at specified intervals. Exemplary specified intervals may include, but are not limited to, administration at intervals of every 24 hours, every 48 hours, and every 72 hours. Alternatively, specified intervals may include, but are not limited to, administration at intervals of at least every 24 hours, at least every 48 hours, and at least every 72 hours. In an aspect of this embodiment, the discontinuous dosing regimen may be continued for a period of time selected from the group consisting of about 1 week, about 2 weeks, about 3 weeks, about 4 weeks, about 5 weeks, about 6 weeks, about 7 weeks, about 8 weeks, about 9 weeks, about 10 weeks, about 11 weeks, and about 12 weeks. In an aspect of this embodiment, the specified interval of administration may be at least once per week. In an aspect of this embodiment, the specified interval of administration may be at least once per week and the discontinuous dosing regimen may continue for a period of time selected from the group consisting of about 1 month, about 2 months, and about 3 months.

In an embodiment of the invention, an effective amount of a GGF2 may be between about 0.001 mg/kg of body weight to about 5 mg/kg of body weight. In an aspect of this embodiment, an effective amount may be between about 0.001 mg/kg of body weight to about 2.5 mg/kg of body weight. In an aspect of this embodiment, an effective amount may be between about 0.001 mg/kg of body weight to about 1.5 mg/kg of body weight. In an aspect of this embodiment, an effective amount may be between about 0.001 mg/kg of body weight to about 0.7 mg/kg of body weight. In an aspect of this embodiment, an effective amount may be between about 0.001 mg/kg of body weight to about 0.5 mg/kg of body weight. In an aspect of this embodiment, an effective amount may be between about 0.001 mg/kg of body weight to about 0.3 mg/kg of body weight. In an aspect of this embodiment, an effective amount may be between about 0.001 mg/kg of body weight to about 0.1 mg/kg of body weight. In an aspect of this embodiment, an effective amount may be between about 0.001 mg/kg of body weight to about 0.05 mg/kg of body weight. In an aspect of this embodiment, an effective amount may be between about 0.001 mg/kg of body weight to about 0.02 mg/kg of body weight.

The actual dosage amount of a composition of the present invention administered to a subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

Certain aspects of the disclosed methods include administration of about 1, 2, 3, 4, 5, 6, 7, 8, 9, 10, 1-10, 1-20, 10-20, 1-30, 1-40, 1-50, 10-20, 10-30, 10, 15, 20, 25, 30, 35, 40, 50, 15-25, 15-40, 15-35, 15-50, 20-50, 20-40, 20-40, 25-35, 30-50, 30-60, 50-75, 50-100, 100, 1-100, 100-150, 150-200, 200, 1-200 µg or mg of neuregulin polypeptide or peptide based on the activity of the particular neuregulin used, route of administration, method of administration and medical context. Certain aspects include the administration of neuregulin prior to and/or after injury. A neuregulin may be administered 12, 24, 48, or 72 hours prior to injury, during injury, and/or at specified or regular intervals following injury. In certain circumstances, a neuregulin may be administered 24 or 48 hours prior to injury, and once every day (*e*.*g*. 24 hours) or every week following injury. Administration prior to injury may be systemic and/or local. Administration during injury may be systemic and/or local. Administration after injury may be systemic and/or local.

Certain aspects of the disclosed methods include administration of about 0.005, 0.1, 0.2, 0.3, 0.4, 0.5, 0.6, 0.7, 0.8, 0.9, 1, 2, 3, 4, 5, 6, 7, 8, 9, 10, 11, 12, 13, 14, or 15 µg or mg, or any value in between, of neuregulin polypeptide or peptide based on the activity of the neuregulin used, route of administration, method of administration and the medical context. For instance, a neuregulin or NRG-1 isoform, including, but not limited to, GGF2 is administered at 0.5, 2.5, 5, 7.5, 10, 12.5, or 15 µg/kg or mg/kg. Any appropriate route of administration may be employed, including, but not limited to, parenteral, subcutaneous, intramuscular, perineural, intracranial, intraorbital, ophthalmic, intraventricular, intracapsular, intraspinal, intracisternal, intraperitoneal, intranasal, aerosol, oral, or transdermal or topical administration (e.g., by applying a device or an adhesive patch carrying a formulation capable of crossing the dermis and entering the bloodstream).

In one aspect of the disclosure, GGF2 dose levels range from about 0.001 mg/kg to 1.5 mg/kg, from about 0.001 mg/kg to 2.5 mg/kg, from about 0.001 mg/kg to 5.0 mg/kg, from about 0.001 mg/kg to 10 mg/kg, or from about 0.001 mg/kg to 15 mg/kg. In a particular embodiment, GGF2 dose levels range from about 0.001 mg/kg to about 0.02 mg/kg, from about 0.02 mg/kg to 0.06 mg/kg, from about 0.06 mg/kg to about 0.1 mg/kg, from about 0.1 mg/kg to about 0.3 mg/kg, from about 0.3 mg/kg to about 0.5 mg/kg, from about 0.5 mg/kg to about 0.7 mg/kg, from about 0.5 mg/kg to about 1.0 mg/kg, from about 0.7 mg/kg to about 1.0 mg/kg, from about 1.0 mg/kg to about 1.5 mg/kg, from about 1.5 mg/kg to about 2 mg/kg, from about 2 mg/kg to about 2.5 mg/kg, from about 2.5 mg/kg to about 3 mg/kg, from about 3 mg/kg to about 3.5 mg/kg, from about 3.5 mg/kg to about 4 mg/kg, from about 4 mg/kg to about 4.5 mg/kg, from about 4.5 mg/kg to about 5 mg/kg, from about 0.001 mg/kg to about 1.5 mg/kg, or from about 0.001 mg/kg to about 5.0 mg/kg. In another particular embodiment, GGF2 dose levels range from about 1 mg/kg to about 15 mg/kg.

In one aspect of the disclosure, GGF2 dose levels range from about 0.001 mg/kg to 1.5 mg/kg, from about 0.001 mg/kg to 2.5 mg/kg, from about 0.001 mg/kg to 5.0 mg/kg, from about 0.001 mg/kg to 10 mg/kg, or from about 0.001 mg/kg to 15 mg/kg, administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months. In a particular embodiment, GGF2 dose levels range from about 0.001 mg/kg to about 0.02 mg/kg, from about 0.02 mg/kg to 0.06 mg/kg, from about 0.06 mg/kg to about 0.1 mg/kg, from about 0.1 mg/kg to about 0.3 mg/kg, from about 0.3 mg/kg to about 0.5 mg/kg, from about 0.5 mg/kg to about 0.7 mg/kg, from about 0.5 mg/kg to about 1.0 mg/kg, from about 0.7 mg/kg to about 1.0 mg/kg, from about 1.0 mg/kg to about 1.5 mg/kg, from about 1.5 mg/kg to about 2 mg/kg, from about 2 mg/kg to about 2.5 mg/kg, from about 2.5 mg/kg to about 3 mg/kg, from about 3 mg/kg to about 3.5 mg/kg, from about 3.5 mg/kg to about 4 mg/kg, from about 4 mg/kg to about 4.5 mg/kg, from about 4.5 mg/kg to about 5 mg/kg, from about 0.001 mg/kg to about 1.5 mg/kg, or from about 0.001 mg/kg to about 5.0 mg/kg administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months. In another particular embodiment, GGF2 dose levels range from about 1 mg/kg to about 15 mg/kg administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months.

In another aspect of the disclosure, at a dosage range of between 5 mg/kg to 15 mg/kg, inclusive of the endpoints, a neuregulin or NRG-1 isoform may be administered to a subject systemically or locally. For instance, when a NRG1 polypeptide or peptide, including, but not limited to GGF2, is administered at a dosage range of between 5 mg/kg to 15 mg/kg, inclusive of the endpoints, the preferred route of administration may be intravenous, subcutaneous, transdermal, or topical.

A neuregulin may be administered prior to injury, during injury, and/or at specified or regular intervals following injury. In a non-limiting example, a neuregulin may be administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks prior to injury. In another embodiment GGF2 is administered at least once per week for 1 month, 2 months, or 3 months prior to injury. Alternatively, or in addition, a neuregulin may be administered once every 24 hours or every week following injury. Administration prior to injury may be systemic and/or local. Administration during injury may be systemic and/or local. Administration after injury may be systemic and/or local. Some doses of a neuregulin (e.g. a dosage range of between 5 mg/kg to 15 mg/kg, inclusive of the end points) may be administered by a non-systemic or local method, including, but not limited to a subcutaneous injection or implant or transdermal delivery method.

A "local", "localized", or "targeted" method of delivery provides a therapeutically-effective neurgulin to the site of injury to improve recovery or function of the peripheral nerve without substantially increase bloodstream levels of the neuregulin or without substantially penetrating the blood-brain barrier, thereby increasing levels of the neuregulin within the cerebral spinal fluid of the central nervous system.

A neuregulin of the disclosed compositions and methods may be any full-length neuregulin encoded by the NRG 1, 2, 3 or 4 genes. In a further aspect a neuregulin can be any functional segment of a neuregulin polypeptide. The functional segment of a neuregulin may contain an EGF-like domain. A neuregulin can be any peptide encoded by the NRG 1, 2, 3 or 4 genes that binds to and activates ErbB receptors. Alternatively, a neuregulin can be any peptide modified from a wild-type peptide encoded by the NRG 1, 2, 3 or 4 genes, such that the modified peptide binds to and activates ErbB receptors.

Neuregulins and polypeptides containing EGF-like domains of neuregulins may be administered to subjects with a pharmaceutically-acceptable diluent, carrier, or excipient, in unit dosage form. Conventional pharmaceutical practice may be employed to provide suitable formulations or compositions to administer such compositions to patients or experimental animals.

Although intravenous administration may be preferred in certain circumstances, any appropriate route of administration may be employed, including, but not limited to, parenteral, subcutaneous, intramuscular, intracranial, intraorbital, ophthalmic, intraventricular, intracapsular, intraspinal, intracisternal, intraperitoneal, intranasal, aerosol, oral, or transdermal or topical administration (e.g., by applying a device or an adhesive patch carrying a formulation capable of crossing the dermis and entering the bloodstream). Therapeutic formulations may be in the form of liquid solutions or suspensions; for oral administration, formulations may be in the form of tablets or capsules; and for intranasal formulations, in the form of powders, nasal drops, or aerosols.

By "neuregulin-1," "NRG-1," "heregulin" is meant a polypeptide that binds to the ErbB receptors 1, 3 or 4; and by receptor pairing (dimerization) also to ErbB2. In one embodiment of the disclosure the neuregulin is encoded by the p185erbB2 ligand gene described in U.S. Pat. Nos. 5,530,109; 5,716,930; and 7,037, 888. In one embodiment of the disclosure the neuregulin is GGF2 or any subsequence thereof, or any molecule that comprises all or an active part of the sequence of GGF2.

In certain embodiments of the disclosed compositions and methods, pharmaceutical compositions may comprise, for example, at least about 0.1% active compound. In other embodiments of the disclosure, an active compound may comprise between about 2% to about 75% of the weight of the unit, or between about 25% to about 60%, for example, and any range derivable therein. In other non-limiting examples, a dose may also comprise from about 1 microgram(µg)/kg/body weight, about 5 µg/kg/body weight, about 10 µg/kg body weight, about 50 µg/kg body weight, about 100 µg/kg body weight, about 200 µg/kg body weight, about 350 µg/kg body weight, about 500 µg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg mg/kg or more per administration, and any range derivable therein. In non-limiting examples of a derivable range from the numbers listed herein, a range of about 5 mg/kg body weight to about 100 mg/kg body weight, about 5 µg/kg body weight to about 500 mg/kg body weight, etc., can be administered, based on the numbers described above.

The term "therapeutically effective amount" or an "effective amount" is intended to mean that amount of neuregulin that elicits a biological or medical response of a tissue, a system, animal or human that is being sought by a researcher, veterinarian, medical doctor or other clinician.

A therapeutic change may be a change in a measured biochemical or physiological characteristic in a direction that alleviates the disease or condition being addressed, e.g., peripheral nerve injury. More particularly, an "effective amount" is an amount sufficient to decrease the symptoms associated with a medical condition or infirmity, to normalize body functions in disease or disorders that result in impairment of specific bodily functions, or to provide improvement in one or more of the clinically measured parameters of a disease or condition. A therapeutically-effective amount or dosage of a neuregulin disclosed herein may be an amount sufficient to restore a measurement of function equivalent to the subject's pre-injury state or to a value associated with normal function. Alternatively, or in addition, a therapeutically-effective amount or dosage of a neuregulin disclosed herein may be an amount sufficient to preserve, restore, or induce survival, connectivity, signal transduction, or function of a sciatic nerve that is either injured or at risk of injury. A therapeutically-effective amount or dosage of a neuregulin disclosed herein may be an amount sufficient to preserve, restore, or induce survival or function of target cell or cells to which a sciatic nerve that is either injured or at risk of injury signals, controls, or innervates.

The use of the term "or" in the claims is used to mean "and/or" unless explicitly indicated to refer to alternatives only or where the alternatives are mutually exclusive." It is also contemplated that anything listed using the term "or" may also be specifically excluded from the other options being set forth.

Throughout this application, the term "about" is used to indicate that a value that is within 85%, 90%, 95% or the standard deviation of error for the device or method being employed to determine the value.

Following long-standing patent law, the words "a" and "an," in the claims or specification, denotes one or more, unless specifically noted.

In certain aspects of the disclosure, neuregulin is used prophylactically thereby preventing or lessening a potential injury. Alternatively, or in addition, neuregulin is used prognostically to indicate the future status of the subject. Alternatively, or further in addition, neuregulin is used diagnostically to indicate the presence or likely presence of a condition or state. Neuregulin may be used therapeutically in order to affect a condition in some way that lessens or removes a symptom or sign of the condition or disease being treated.

Neuregulins play an important role in the adult peripheral nervous system. For example, neuregulins, specifically glial growth factor 2 (GGF2), influence multiple aspects of Schwann cell differentiation, including the survival of Schwann cell precursors, Schwann cell proliferation, axon ensheathment, and myelination. In addition, neuregulins also play a role in regulating acetylcholine receptors at the neuromuscular junction. Neuregulins also influence Schwann cell function in peripheral nerves, and have myogenic and metabolic effects in skeletal muscle. As such, the goal of the study presented herein was to evaluate the effects of the neuregulin 1 isoform glial growth factor 2 (GGF2) on microscopic changes in the skeletal muscle and sciatic nerve function and recovery thereof following sciatic nerve crush injury.

The data presented herein demonstrate that GGF2 benefits peripheral nerve myelination, target muscle function, and sensory functions of the nerve following peripheral nerve injury (for example, following sciatic nerve injury). Specifically, as described herein, histological evaluation of the gastrocnemius muscle demonstrated an increase in fibrosis in crush animals compared to sham at 2, 4, and 6 weeks following injury; however, GGF2 treatment significantly reduced injury-induced fibrosis to near normal levels at 6 weeks post injury. Moreover, a significant reduction was seen in myofiber area at 2, 4, and 6 weeks following injury; however, GGF2 treatment promoted a shift in the myofiber area towards increased size (i.e., more like sham controls) at 4 and 6 weeks post injury. Following injury, a wide variety of lesions with varying severities were observed in the sciatic nerve compared to sham animals; however, GGF2 qualitatively reduced the degree of demyelination compared to vehicle-treated animals in the lesioned sciatic nerve. GGF2 mitigated signs of mechanical and cold hypersensitivity following crush injury, while enhancing mechanical and cold sensory functions following sciatic deafferentation by transection.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present disclosure and are included to demonstrate certain non-limiting aspects of the present disclosure. The disclosure may be better understood by reference to one of these drawings in combination with the detailed description of specific embodiments presented herein.

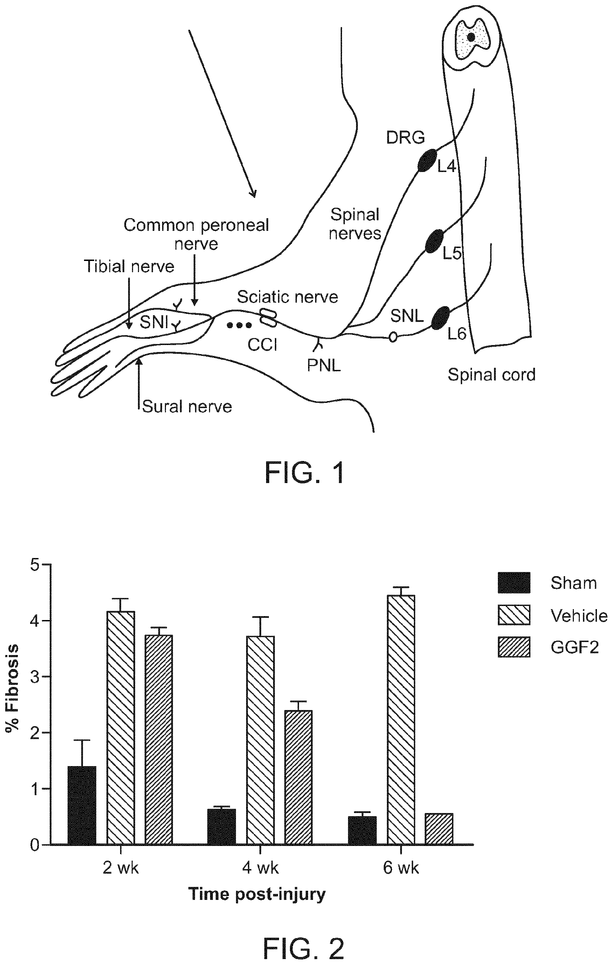

Figure 1 is a schematic illustration of sciatic nerve crush injury 8-10 mm proximal to the sciatic nerve trifurcation.
Figure 2 is a graph depicting the percent change in fibrosis in the lateral gastrocnemius muscle observed as a function of time (2, 4, or 6 weeks) under conditions of sham injury, vehicle treatment following crush injury, or GGF2 treatment following crush injury.

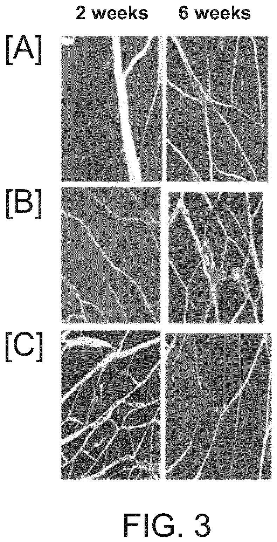

Figure 3 is a series of photomicrographs (Trichrome 100X) of representative muscle sections from sham [A], vehicle-treated [B], and GGF2-treated animals [C] at 2 and 6 weeks following injury.

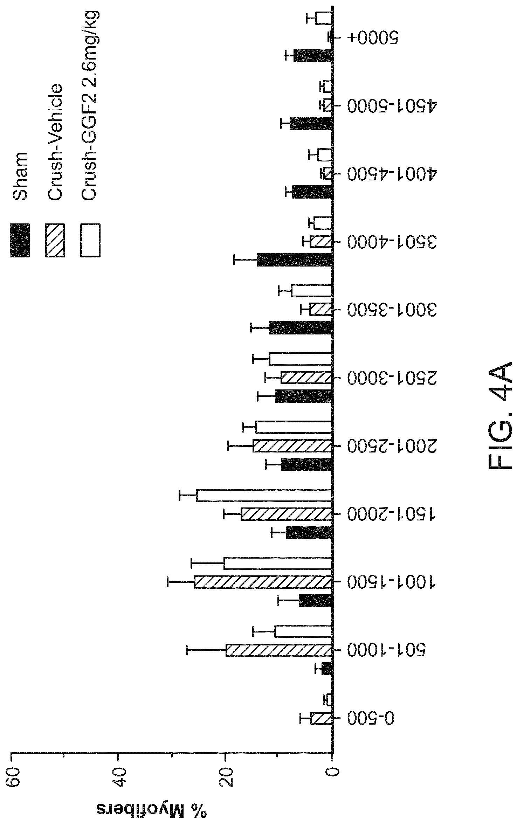

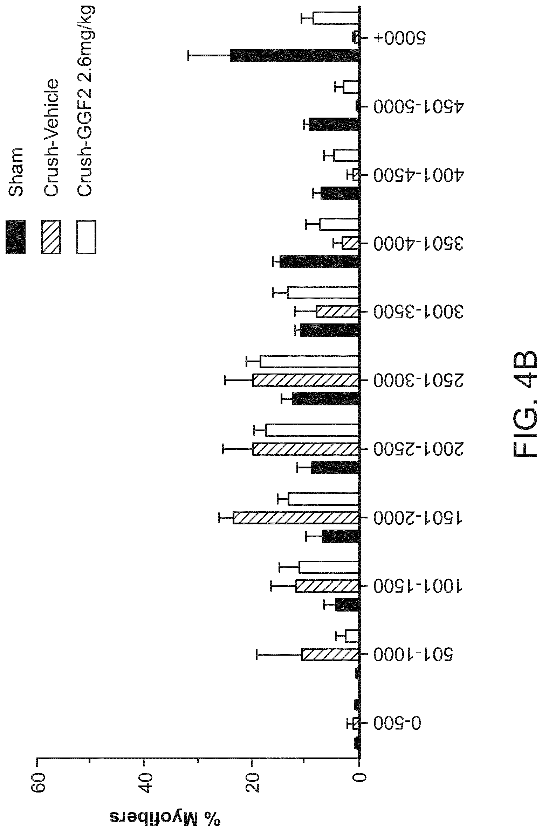

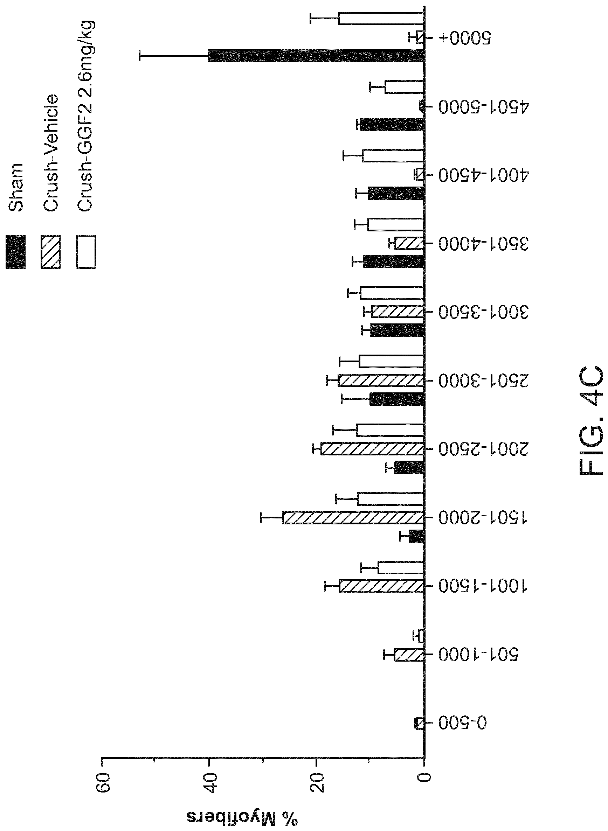

Figures 4A, 4B, and 4C are a series of graphs depicting the percent fibrosis observed as a function of time (2, 4, or 6 weeks, respectively) under conditions of sham injury, vehicle treatment following crush injury, or GGF2 treatment following crush injury.

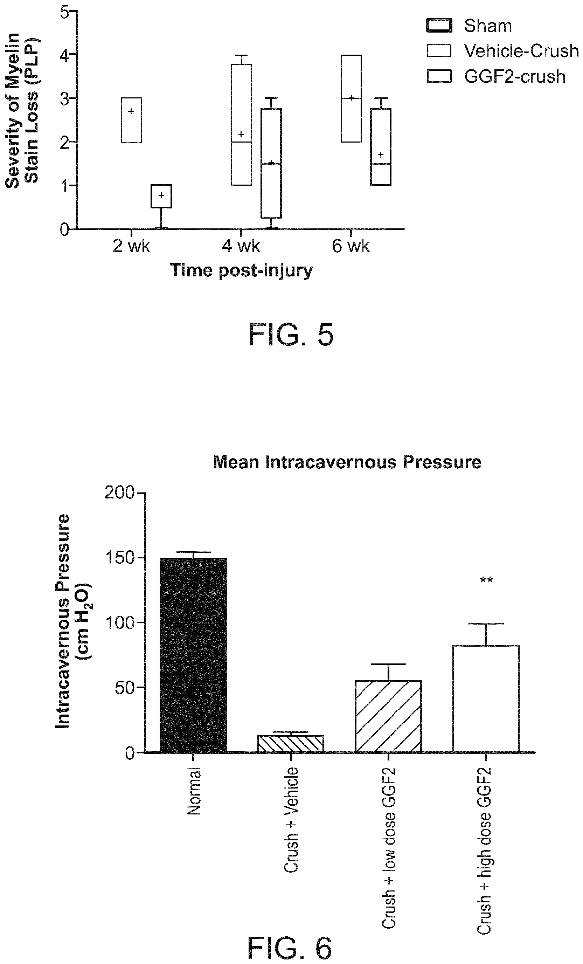

Figure 5 is a graph showing an assessment of myelination in the sciatic nerve proteolipid protein stain (PLP) at 2, 4, and 6 weeks under conditions of sham injury, vehicle treatment following crush injury, or GGF2 treatment following crush injury.
Figure 6 is a graph depicting the mean intracavernous pressure (ICP) change from baseline as a function of experimental condition. "Low dose" GGF2 = 0.5 mg/kg and "high dose" 0.54 GGF2 5 mg/kg ("high dose"). 32 male 3 month old Sprague-Dawley rats split into four experimental conditions: Group 1 - sham surgery, Group 2 - crush injury + no GGF2, Group 3 - crush + low dose GGF2 (0.5 mg/kg), and Group 4 - crush + high dose GGF2 (5.0 mg/kg). GGF2 delivered 24 hrs pre-crush, 24 hrs post-crush and one every 7 days (once a week) until study end (5 weeks) post-surgery. Surgery included a lower midline incision to expose prostate, cavernous nerves and major pelvic ganglia (MPG) and 2 minute crush injury (needle driver at 90 degree angle and 'one-click' pressure).

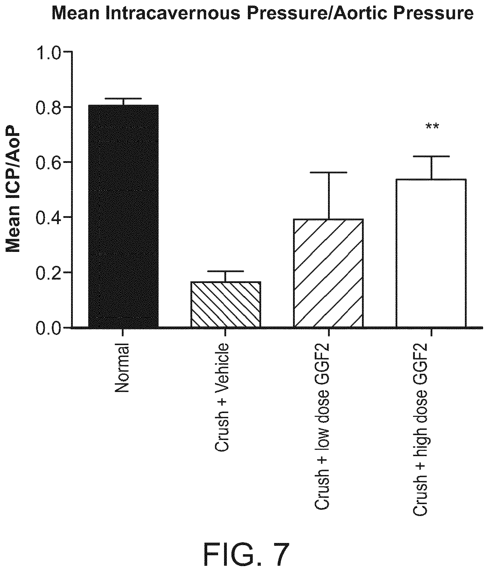

Figure 7 is a graph depicting the mean ICP normalized to the mean Aortic Pressures (MAP) as a function of experimental condition. ICP/MAP ratios are 0.81 (normal), 0.15 (crush and no treatment) versus 0.4 GGF2 0.5 mg/kg ("low dose") and 0.54 GGF2 5 mg/kg ("high dose") (all values p<0.05). 32 male 3 month old Sprague-Dawley rats split into four experimental conditions: Group 1 - sham surgery, Group 2 - crush injury + no GGF2, Group 3 - crush + low dose GGF2 (0.5 mg/kg), and Group 4 - crush + high dose GGF2 (5.0 mg/kg). GGF2 delivered 24 hrs pre-crush, 24 hrs post-crush and one every 7 days (once a week) until study end (5 weeks) post-surgery. Surgery included a lower midline incision to expose prostate, cavernous nerves and major pelvic ganglia (MPG) and 2 minute crush injury (needle driver at 90 degree angle and 'one-click' pressure).

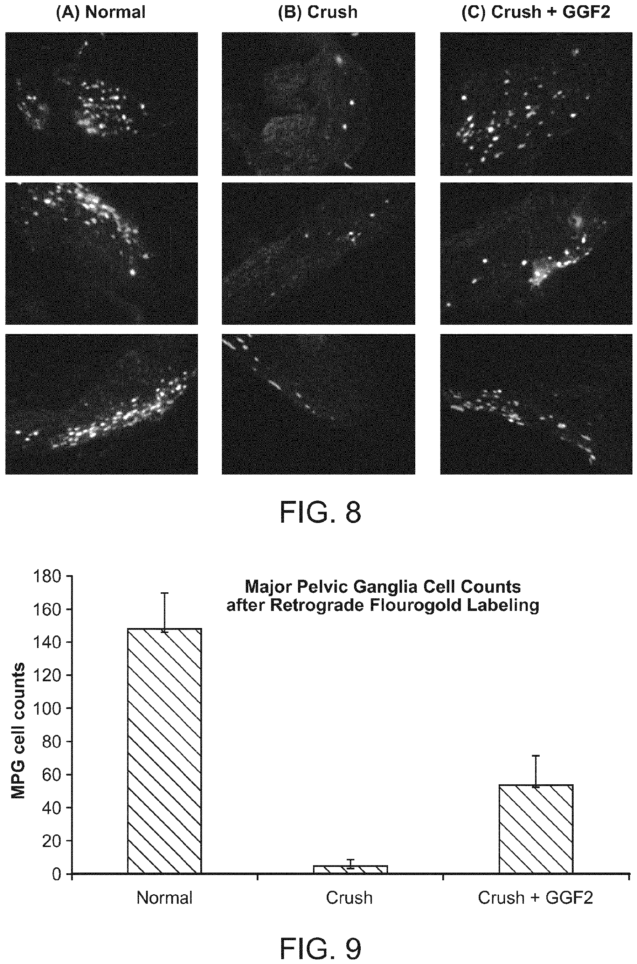

Figure 8A-C is a series of photographs depicting representative Fluoro-gold labeling of major pelvic ganglia (MPG) from 3 animals per treatment group ((panel A) normal, (panel B) crush, (panel C) crush+ GGF2). Fluoro-gold injected into the penile tissue is transported retrogradely back through intact nerves to the cell bodies in the MPG. Panel A: Normal animals demonstrate the amount of retrograde labeling observed in the absence of nerve injury. Panel B: Crush animals demonstrate the dramatic reduction in intact nerve fibers from the injury, as the fluoro-gold label is not able to be transported all the way back to the MPG. Panel C: Crush+GGF2 animals show an increased number of fluoro-gold labeled MPG cells, indicating that there are more preserved nerve fibers present after injury as a result of GGF2 treatment.
Figure 9 is a graph depicting the quantitation of fluoro-gold labeling in the MPG. Results showed that normal animals have a large number of cell bodies labeling in the MPG. Following a crush injury the number of labeled cells is dramatically reduced, consequent to nerve fiber damage and the resulting inability to transport retrogradely the label back to the MPG. However, GGF2 treatment improved the number of intact nerve fibers available to transport the fluoro-gold from the penile tissue to the MPG in a retrograde manner, resulting in a larger number of labeled cells.

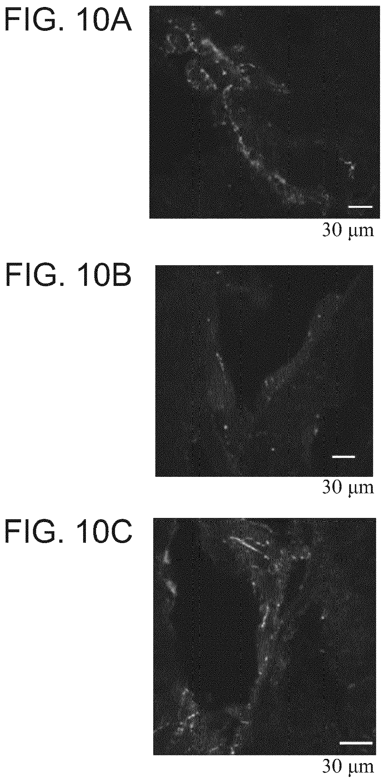

Figure 10A-C is a series of photographs depicting representative staining of nNos levels. Cavernous nNOS is a well-established marker of cavernous nerve preservation. Results of this work included normal tissue staining (panel A). By comparison, there was a significant loss of nNOS staining after cavernous nerve crush injury (panel B). Preserved nNOS staining of cavernous nerve endings in the penile corpora demonstrated increased rates of survival of cavernous nerves following crush injury with GGF2 treatment (panel C). Density of staining indicates preservation of nNOS staining with GGF2 treatment.

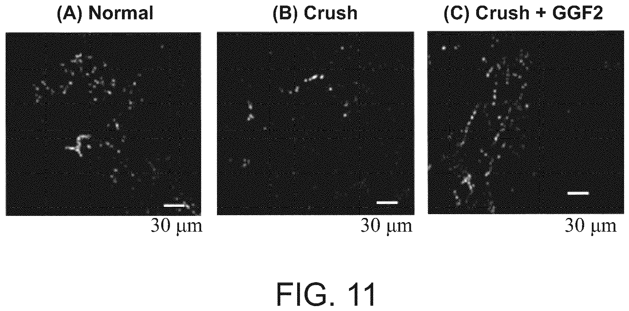

Figure 11A-C is a series of photographs depicting representative staining of tyrosine hydroxylase (TH) levels. Panel A depicts normal tissue staining and on panel B a significant loss of TH staining after cavernous nerve crush injury. Panel C shows preserved TH staining of cavernous nerve endings in the penile corpora; this finding corresponds to a general preservation or reestablishment of penile innervation following crush injury being produced by GGF2 treatment. Thus, the density of staining indicated preservation of TH staining with GGF2 treatment.

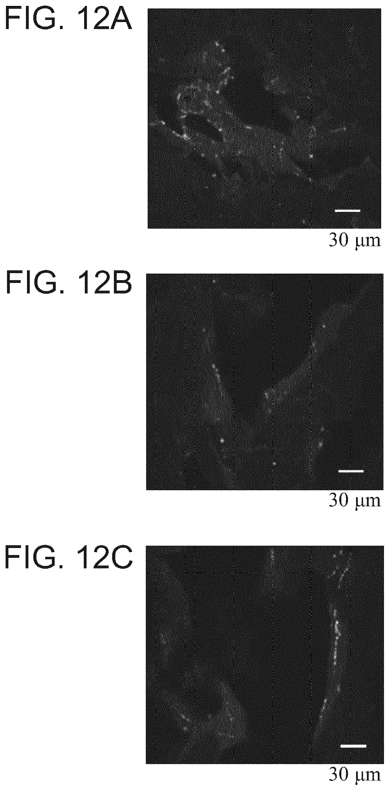

Figure 12A-C is a series of photographs depicting representative staining of vesicular acetylcholine transporter (VaChT). Results show normal tissue staining (panel A), and a significant loss of VaChT staining after cavernous nerve crush injury (panel B). In contrast, the preserved VaChT staining of cavernous nerve endings in the penile corpora shown in (panel C) demonstrates increased rates of survival of cavernous nerves following crush injury with GGF2 treatment (C). Density of staining shows trends towards preservation of VaChT staining with GGF2 treatment.

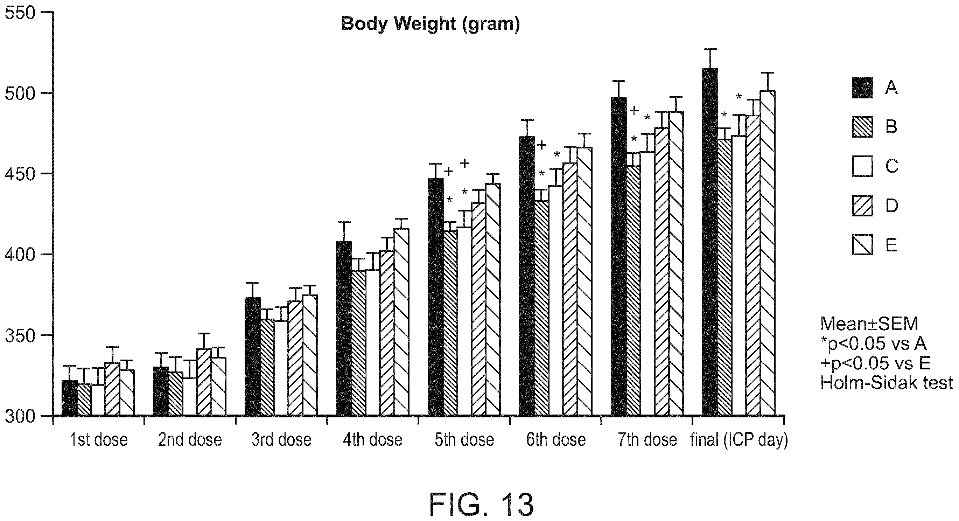

Figure 13 is a graph depicting the change in body weight of study subjects as a function of progressive number of doses of GGF2 or vehicle (see Example 6).

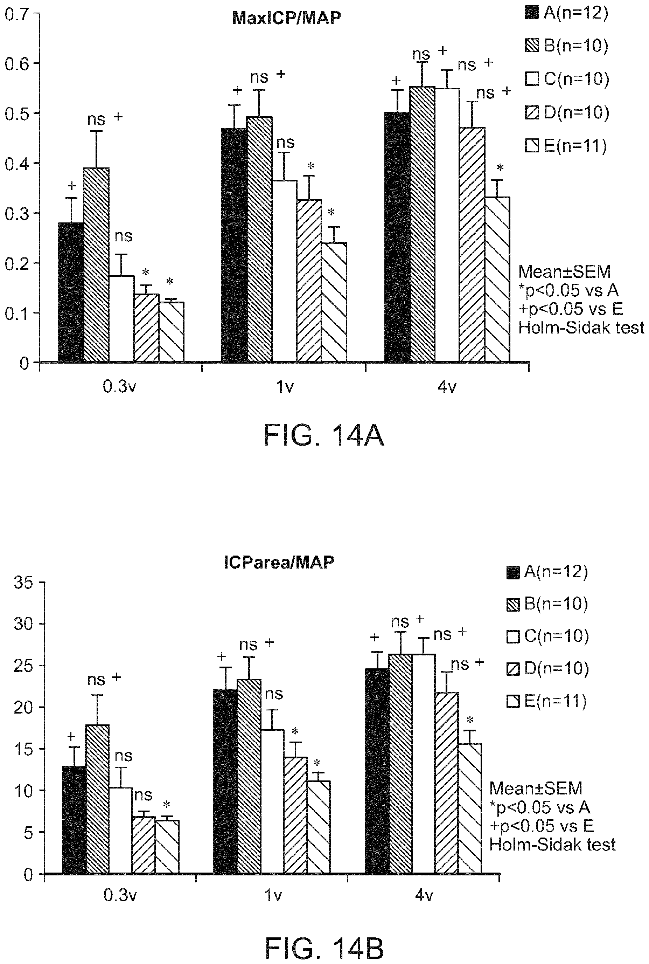

Figure 14A is a graph depicting the change in intracavernous pressure (Maximum ICP normalized to mean aortic pressure (MAP)) at varying levels of electrical stimulation of the cavernous nerve (see Example 6). ICP response was significantly higher in Groups C and D. Group A: sham + vehicle. Group B: sham + GGF2. Group C: BCI+ GGF2 (5 mg/kg, sc). Group D: BCI+ GGF2 (15 mg/kg, sc). Group E: BCI+ vehicle.
Figure 14B is a graph depicting the change in intracavernous pressure (ICParea/MAP) at varying levels of electrical stimulation of the cavernous nerve (see Example 6). ICP response was significantly higher in Groups C and D. Group A: sham + vehicle. Group B: sham + GGF2. Group C: BCI+ GGF2 (5 mg/kg, sc). Group D: BCI+ GGF2 (15 mg/kg, sc). Group E: BCI+ vehicle.

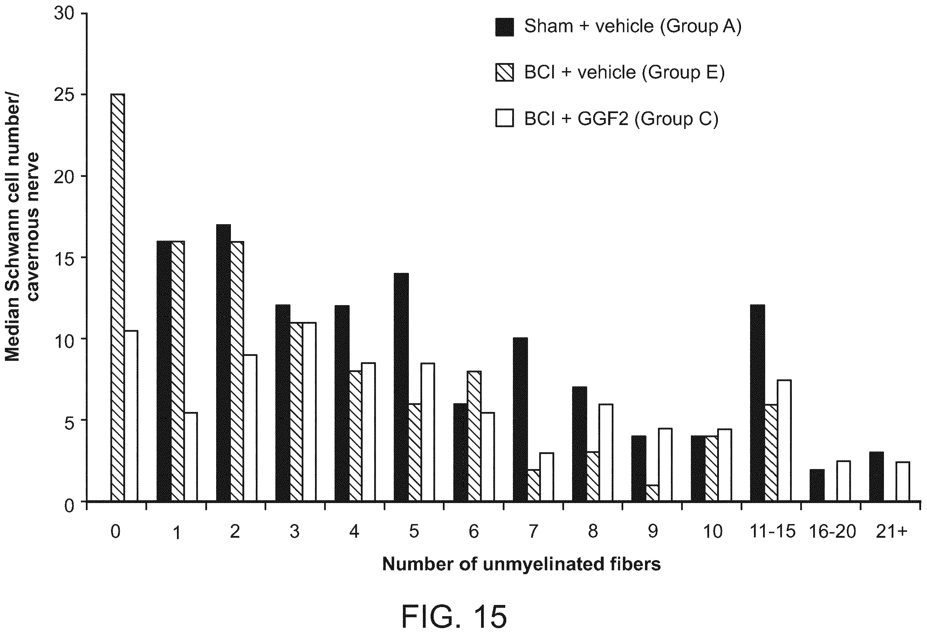

Figure 15 is a graph depicting the median Schwann cell number per cavernous nerve as a function of the number of unmyelinated fibers for treatment groups A, E, and C (see Example 6).

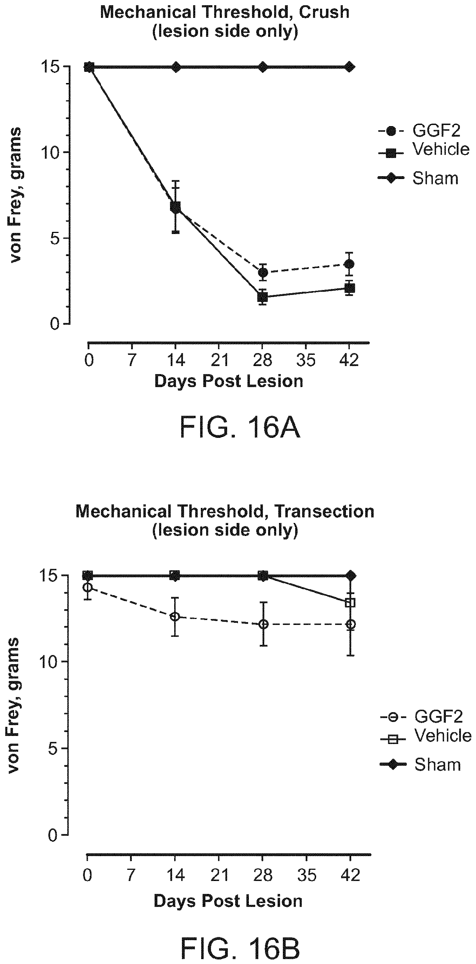

Figure 16A-B is a pair of graphs depicting a time course of von Frey mechanical (tactile) thresholds over 6 weeks of study under conditions of sham injury, vehicle treatment of GGF2 treatment following crush injury (panel A) or transection injury (panel B).

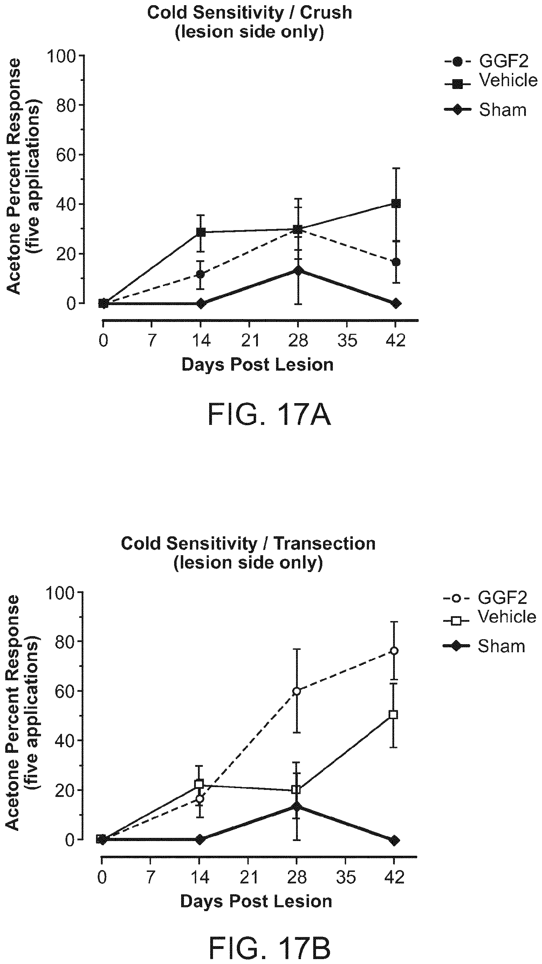

Figure 17A-B is a pair of graphs depicting a time course of behavioral responses to acetone evaporative cooling over 6 weeks of study under conditions of sham injury, vehicle treatment or GGF2 treatment following crush injury (panel A) or transection injury (panel B).

### DETAILED DESCRIPTION

Injury to peripheral nerves is the common result of various events, compression, contusion, transection, crush or stretch, caused, e.g., by trauma, accident or surgery. While the external factors leading to the nerve injury are varied, the manifestations at the nerve level have common features (For review see e.g., Lee and Wolfe, J Am Acad Orthop Surg, 8(4), p. 243, 2008). Traumatic injury of any etiology often causes damage to myelination, epineurium, perineurium, endoneurium and axons. In the mildest of cases, injury is primarily to the myelin and epineurium whereafter complete recovery occurs spontaneously within several days or weeks.

Many nerve injuries result in disruption of endoneurium and axons as well as disruption of function that either does not fully recover or recovers over a prolonged period of time.

With respect to a peripheral nerve injury that involves damage to an axon, there is local degeneration of that axon that occurs within hours after the injury. Over the next few days, the proximal neuron cell body and axon undergo a process known as Wallerian degeneration. Following degeneration of the axon, the myelin-producing Schwann cell dies leaving debris and inflammation. This Schwann cell death and related inflammation exacerbate nerve damage.

Unlike the central nervous system, a significant amount of regeneration can occur in peripheral nerves. The axons grow along the perineurium channels and re-innervate distal targets, and Schwann cells remyelinate axons. Although there is regeneration of peripheral nerves, the natural process of regeneration mediated by the body does not provide full restoration, and many neurons that undergo degeneration never regenerate or never find their original target and permanent dysfunction(s) results. This dysfunction can comprise loss of motor function, loss of sensory function, parathesias, loss of reflexes, rigidity, contractures, or decreased range of motion.

Pain is normally associated with sensory nerve injury or damage, and results in guarding and immobilization of the affected area. Nociception (the neuronal signaling underlying the sensation of pain), therefore, is concomitant to mechanisms for and the promotion of rapid healing, albeit triggering an unpleasant sensory and emotional experience. However, in many pathological situations, nociceptive inputs can result in functional changes that are actively detrimental to the organism.

Nerve injury results in the alteration of many of the properties of primary afferent neurons and their central connections in the spinal cord, leading to allodynia (the perception of pain from a normally innocuous stimulus), hyperalgesia (an exaggerated response to any given pain stimulus) and an expansion of the receptive field (i.e. the area that is "painful" when a stimulus is applied). The majority of chronic pain conditions arise as a result of damage to either central or peripheral nervous tissue.

A body of literature demonstrates that neuregulins enhance the ability of neurons to regenerate through artificial conduits and function as an adjunctive therapy with cell therapies such as Schwann cell grafts. Prior to the present disclosure, it was not known that neuregulins alone could treat, such as by protecting and/or restoring function) in peripheral nerve injury.

### Neuregulin Compositions

Neuregulin may be used prophylactically, thereby preventing or minimizing a potential injury. Alternatively, or in addition, neuregulin may be used to treat an injury. The term treating may include, but is not limited to, prevention of further injury, inhibiting further or ongoing damage, ameliorating a sign or symptom of injury, and/or improving/restoring function following injury.

A neuregulin may be administered to a subject at risk of incurring a peripheral nerve injury or having an existing peripheral nerve injury to improve muscle function, such as, by way of example, and not limitation, skeletal muscle function. Examples of improved muscle function include, but are not limited to, increase in muscle mass (e.g., increase in myofiber size), muscle strength, reduction in muscle atrophy and/or a reduction in muscle fibrosis. In a preferred aspect of the compositions and methods of the disclosure, the neuregulin is GGF2.

A neuregulin may be administered to a subject at risk of incurring a peripheral nerve injury or having an existing peripheral nerve injury to treat a sensory and/or motor deficit and/or hypersensitivity. Nonlimiting examples of sensory deficits and/or hypersensitivity that may be treated by the methods of the disclosure include diabetic neuropathy, traumatic neuropathy and/or chemotherapeutic neuropathy (for example, following administration of cisplatin or pacitaxel). In a preferred aspect, the neuregulin is GGF2.

A neuregulin may be administered a subject at risk of incurring a peripheral nerve injury or having an existing peripheral nerve injury to treat a dysfunction of the autonomic nervous system in a subject having an existing peripheral nerve injury. Exemplary dysfunctions in the autonomic nervous system include dysfunctions in the sympathetic and/or parasympathetic nervous systems. Nonlimiting examples of autonomic dysfunctions that may be treated by the methods of the disclosure include dysfunctions in regional blood flow (e.g. cavernous nerve injury resulting in erectile dysfunction), gut motility secretions, respiratory changes, bladder changes and/or ocular changes. In a preferred aspect, the neuregulin is GGF2.

Exemplary compositions include, but are not limited to, a neuregulin, a NRG-1, or a GGF2 nucleic acid or peptide molecule. Exemplary peripheral nerves subject to the disclosed methods include, but are not limited to, a sciatic nerve and/or a cavernous nerve.

The actual dosage amount of a composition of the present invention administered to a subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

Certain aspects of the disclosed methods include administration of about 1, 2, 3, 4, 5, 6, 7, 8, 9, 10, 1-10, 1-20, 10-20, 1-30, 1-40, 1-50, 10-20, 10-30, 10, 15, 20, 25, 30, 35, 40, 50, 15-25, 15-40, 15-35, 15-50, 20-50, 20-40, 20-40, 25-35, 30-50, 30-60, 50-75, 50-100, 100, 1-100, 100-150, 150-200, 200, 1-200 µg or mg of neuregulin polypeptide or peptide based on the activity of the particular neuregulin used, route of administration, method of administration and medical context. Certain aspects include the administration of neuregulin prior to and/or after injury.

A neuregulin may be administered prior to injury, during injury, and/or at specified or regular intervals following injury. In a non-limiting example, a neuregulin may be administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks prior to injury. In another embodiment GGF2 is administered at least once per week for 1 month, 2 months, or 3 months prior to injury. Alternatively, or in addition, a neuregulin may be administered once every 24 hours or every week following injury. Administration prior to injury may be systemic and/or local. Administration during injury may be systemic and/or local. Administration after injury may be systemic and/or local.

Certain aspects of the disclosed methods include administration of about 0.005, 0.1, 0.2, 0.3, 0.4, 0.5, 0.6, 0.7, 0.8, 0.9, 1, 2, 3, 4, 5, 6, 7, 8, 9, 10, 11, 12, 13, 14, or 15 µg or mg, or any value in between, of neuregulin polypeptide or peptide based on the activity of the neuregulin used, route of administration, method of administration and the medical context. For instance, a neuregulin or NRG-1 isoform, including, but not limited to, GGF2 is administered at 0.5, 2.5, 5, 7.5, 10, 12.5, or 15 µg/kg or mg/kg. Any appropriate route of administration may be employed, including, but not limited to, parenteral, subcutaneous, intramuscular, perineural, intracranial, intraorbital, ophthalmic, intraventricular, intracapsular, intraspinal, intracisternal, intraperitoneal, intranasal, aerosol, oral, or transdermal or topical administration (*e.g*., by applying a device or an adhesive patch carrying a formulation capable of crossing the dermis and entering the bloodstream).

In one aspect of the disclosure, GGF2 dose levels range from about 0.001 mg/kg to 1.5 mg/kg, from about 0.001 mg/kg to 2.5 mg/kg, from about 0.001 mg/kg to 5.0 mg/kg, from about 0.001 mg/kg to 10 mg/kg, or from about 0.001 mg/kg to 15 mg/kg, administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months. In a particular embodiment, GGF2 dose levels range from about 0.001 mg/kg to about 0.02 mg/kg, from about 0.02 mg/kg to 0.06 mg/kg, from about 0.06 mg/kg to about 0.1 mg/kg, from about 0.1 mg/kg to about 0.3 mg/kg, from about 0.3 mg/kg to about 0.5 mg/kg, from about 0.5 mg/kg to about 0.7 mg/kg, from about 0.5 mg/kg to about 1.0 mg/kg, from about 0.7 mg/kg to about 1.0 mg/kg, from about 1.0 mg/kg to about 1.5 mg/kg, from about 1.5 mg/kg to about 2 mg/kg, from about 2 mg/kg to about 2.5 mg/kg, from about 2.5 mg/kg to about 3 mg/kg, from about 3 mg/kg to about 3.5 mg/kg, from about 3.5 mg/kg to about 4 mg/kg, from about 4 mg/kg to about 4.5 mg/kg, from about 4.5 mg/kg to about 5 mg/kg, from about 0.001 mg/kg to about 1.5 mg/kg, or from about 0.001 mg/kg to about 5.0 mg/kg administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months. In another particular embodiment, GGF2 dose levels range from about 1 mg/kg to about 15 mg/kg administered about every 24 hours (or once per day), at least every 24 hours, every 48 hours, at least every 48 hours, at least once every other day, every 72 hours, at least every 72 hours, for about 1 week, 2 weeks, 3 weeks, 4 weeks, 5 weeks, 6 weeks, 7 weeks, 8 weeks, 9 weeks, 10 weeks, 11 weeks, or 12 weeks. In another embodiment GGF2 is administered at least once per week for 1 month, 2 months, or 3 months.

In another aspect of the disclosure, at a dosage range of between 5 mg/kg to 15 mg/kg, inclusive of the endpoints, a neuregulin or NRG-1 isoform may be administered to a subject systemically or locally. For instance, when a NRG1 polypeptide or peptide, including, but not limited to GGF2, is administered at a dosage range of between 5 mg/kg to 15 mg/kg, inclusive of the endpoints, the preferred route of administration may be intravenous, subcutaneous, transdermal, or topical. A neuregulin may be administered prior to injury, during injury, and/or at specified or regular intervals following injury. In one example, a neuregulin may be administered 24 or 48 hours prior to injury and once every 24 hours or every week following injury. Administration prior to injury may be systemic and/or local. Administration during injury may be systemic and/or local. Administration after injury may be systemic and/or local. Some doses of a neuregulin (e.g. a dosage range of between 5 mg/kg to 15 mg/kg, inclusive of the end points) may be administered by a non-systemic or local method, including, but not limited to a subcutaneous injection or implant or transdermal delivery method.

In certain embodiments of the disclosed compositions and methods, pharmaceutical compositions may comprise, for example, at least about 0.1% active compound. In other embodiments, an active compound may comprise between about 2% to about 75% of the weight of the unit, or between about 25% to about 60%, for example, and any range derivable therein. In other non-limiting examples, a dose may also comprise from about 1 microgram(µg)/kg/body weight, about 5 µg/kg/body weight, about 10 µg/kg body weight, about 50 µg/kg body weight, about 100 µg/kg body weight, about 200 µg/kg body weight, about 350 µg/kg body weight, about 500 µg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg mg/kg or more per administration, and any range derivable therein. In non-limiting examples of a derivable range from the numbers listed herein, a range of about 5 mg/kg body weight to about 100 mg/kg body weight, about 5 µg/kg body weight to about 500 mg/kg body weight, etc., can be administered, based on the numbers described above.

The term "about" is used to indicate that a value that is within 85%, 90%, 95% or the standard deviation of error for the device or method being employed to determine the value.

Neuregulins and polypeptides containing EGF-like domains of neuregulins can be administered to subjects with a pharmaceutically-acceptable diluent, carrier, or excipient, in unit dosage form. Conventional pharmaceutical practice may be employed to provide suitable formulations or compositions to administer such compositions to patients or experimental animals. Although intravenous administration is preferred, any appropriate route of administration may be employed, for example, parenteral, subcutaneous, intramuscular, perineural, intracranial, intraorbital, ophthalmic, intraventricular, intracapsular, intraspinal, intracisternal, intraperitoneal, intranasal, aerosol, oral, or transdermal or topical administration (e.g., by applying a device or an adhesive patch carrying a formulation capable of crossing the dermis and entering the bloodstream). Therapeutic formulations may be in the form of liquid solutions or suspensions. For oral administration, formulations may be in the form of tablets or capsules. For intranasal formulations, formulations may be in the form of powders, nasal drops, or aerosols.

By "neuregulin-1," "NRG-1," "heregulin" is meant a polypeptide that binds to the ErbB receptors 3 or 4, and by receptor pairing (dimerization) also to ErbB2. The neuregulin may be GGF2 or any subsequence thereof, or any molecule that comprises all or an active part of the sequence of GGF2. The GGF2 may be a wild type sequence, a subsequence, or any variant thereof that comprises all or an active part of the sequence of GGF2. The GGF2 sequence may be a human sequence a subsequence, or any variant thereof that comprises all or an active part of the sequence of GGF2.

A nonlimiting example of a GGF2 amino acid sequence of the disclosure (with a region comprising its EGF-like domain under lined) is:

A neuregulin polypeptide or segment thereof may be 75, 80, 85, 90, 91, 92, 93, 94, 95, 96, 97, 98, 99, or 100% identical or homologous to the amino acid sequence of GGF2. A neuregulin-like polypeptide may be 75, 80, 85, 86, 97, 88, 89, 90, 91, 92, 93, 94, 95, 96, 97, 98, 99, or 100% identical or homologous to the amino acid sequence of the EGF-like domain of GGF2.

The terms "protein" or "polypeptide" refers to a molecule comprising at least ten amino acid residues. The protein may comprise all or part of the GGF2 polypeptide. A wild-type version of a protein or polypeptide may be used. Alternatively, or in addition, a modified protein or polypeptide is used to treat peripheral nerve injury. The terms "peptide," "protein" or "polypeptide" are used interchangeably. The term "peptide" may be used to refer to amino acid sequences of any length.

A "modified peptide" refers to a peptide whose chemical structure, particularly its amino acid sequence, is altered with respect to the respective wild-type peptide. In some embodiments, a modified peptide has at least one modified amino acid. In some embodiments, a modified peptide has at least one d-amino acid. In some embodiments, a modified peptide has at least one non-naturally occurring amino acid.

Modified peptides can include substitutional, insertional, or deletion variants. Deletion variants typically lack one or more residues of the native or wild-type molecule. Individual residues may be deleted or a number of contiguous amino acids can be deleted. A stop codon may be introduced (by substitution or insertion) into an encoding nucleic acid sequence to generate a truncated protein. Insertional mutants typically involve the addition of material at a non-terminal point in the peptide. This may include the insertion of one or more residues. Terminal additions, often called fusion proteins or fusion peptides, may also be generated. Substitutional variants typically contain the exchange of one amino acid for another at one or more sites within the peptide, and may be designed to modulate one or more properties of the peptide, with or without the loss of other functions or properties, such as binding and activation of neuregulin receptors. Substitutions may be conservative, that is, one amino acid is replaced with one of similar shape and charge. Alternatively, substitutions may be non-conservative such that a function or activity of the peptide may be affected. Non-conservative changes typically involve substituting a residue with one that is chemically dissimilar, such as a polar or charged amino acid for a nonpolar or uncharged amino acid, and vice versa.

Conservative substitutions" may include, without limitation, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine or leucine or methionine; serine to threonine; threonine to serine; tryptophan to tyrosine; tyrosine to tryptophan or phenylalanine; and valine to isoleucine or leucine.

Amino acid and nucleic acid sequences may include additional residues, such as additional N- or C-terminal amino acids, or 5' or 3' sequences, respectively, so long as the sequence meets the functional criteria set forth herein such as the maintenance of biological activity. The addition of terminal sequences particularly applies to nucleic acid sequences that may, for example, include various non-coding sequences flanking either of the 5' or 3' portions of the coding region.

The term "therapeutically effective amount" or an "effective amount" is intended to mean that amount of neuregulin that elicits a biological or medical response of a tissue, a system, animal or human that is being sought by a researcher, veterinarian, medical doctor or other clinician.

A therapeutic change may be a change in a measured biochemical or physiological characteristic in a direction that alleviates the disease or condition being addressed, e.g., peripheral nerve injury, muscle atrophy (increases muscle mass, improves muscle strength, reduces muscular atrophy), reduce neuropathic pain, enhance sensory recovery, enhance peripheral nerve remyelination. More particularly, an "effective amount" is an amount sufficient to decrease the symptoms associated with a medical condition or infirmity, to normalize body functions in disease or disorders that result in impairment of specific bodily functions, or to provide improvement in one or more of the clinically measured parameters of a disease or condition.

### Pharmaceutical Formulations

Pharmaceutical formulations of the present disclosure comprise an effective amount of a peptide dissolved or dispersed in a pharmaceutically acceptable carrier. The phrases "pharmaceutical or pharmacologically acceptable" refer to compositions that do not generally produce an adverse, allergic or other untoward reaction when administered to a subject, e.g., a human, as appropriate. The preparation of such pharmaceutical compositions are known to those of skill in the art in light of the present disclosure, as exemplified by Remington's Pharmaceutical Sciences, 18th Ed. Mack Printing Company, 1990. Moreover, for human administration purposes it will be understood that preparations should meet sterility, pyrogenicity, general safety and purity standards as required by, e.g., the USFDA Office of Biological Standards.

Moreover, as used herein "pharmaceutically acceptable carrier" includes materials such as solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as is known to one of ordinary skill in the art in view of the present disclosure. Except insofar as any conventional carrier is incompatible with an active ingredient, its use in the therapeutic or pharmaceutical compositions is contemplated.

The pharmaceuticals of the present disclosure may comprise different types of carriers depending on whether it is to be administered in solid, liquid or aerosol form, and whether it need to be sterile for such routes of administration as injection. Neuregulin polypeptides or peptides of the disclosure can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, intratumorally, intramuscularly, perineurally, subcutaneously, subconjunctival, intravesicularly, mucosally, intrapericardially, intraumbilically, intraocularally, orally, topically, locally, by inhalation (e.g., aerosol). Moreover, neuregulin polypeptides or peptides of the disclosure can be administered by injection, infusion, continuous infusion, localized perfusion bathing target cells directly, via a catheter, via a lavage, or by other method or any combination of the forgoing as would be known to one of ordinary skill in the art.

The actual dosage amount of a composition administered to a subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain embodiments of the disclosed compositions and methods, pharmaceutical compositions may comprise, for example, at least about 0.1% active compound. In other embodiments, an active compound may comprise between about 2% to about 75% of the weight of the unit, or between about 25% to about 60%, for example, and any range derivable therein. In other non-limiting examples, a dose may also comprise from about 1 microgram(µg)/kg/body weight, about 5 µg/kg/body weight, about 10 µg/kg body weight, about 50 µg/kg body weight, about 100 µg/kg body weight, about 200 µg/kg body weight, about 350 µg/kg body weight, about 500 µg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg mg/kg or more per administration, and any range derivable therein. In non-limiting examples of a derivable range from the numbers listed herein, a range of about 5 mg/kg body weight to about 100 mg/kg body weight, about 5 µg/kg body weight to about 500 mg/kg body weight, etc., can be administered, based on the numbers described above.

In any case, the composition may comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof

The pharmaceuticals may be formulated into a composition in a free base, neutral or salt form. Pharmaceutically acceptable salts include the acid addition salts, e.g., those formed with the free amino groups of a peptide composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine.

In embodiments where the composition is in a liquid form, a carrier can be a solvent or dispersion medium comprising but not limited to, water, ethanol, polyol (e.g., glycerol, propylene glycol, liquid polyethylene glycol, etc.), lipids (e.g., triglycerides, vegetable oils, liposomes) and combinations thereof The proper fluidity can be maintained, for example, by the use of a coating, such as lecithin; by the maintenance of the required particle size by dispersion in carriers such as, for example liquid polyol or lipids; by the use of surfactants such as, for example hydroxypropylcellulose; or combinations of such methods. In many cases, it will be preferable to include isotonic agents, such as, for example, sugars, sodium chloride or combinations thereof.

In certain embodiments, the compositions are prepared for administration by such routes as oral ingestion. In these embodiments, the solid composition may comprise, for example, solutions, suspensions, emulsions, tablets, pills, capsules (e.g., hard or soft shelled gelatin capsules), sustained release formulations, buccal compositions, troches, elixirs, suspensions, syrups, wafers, or combinations thereof Oral compositions may be incorporated directly with the food of the diet. Preferred carriers for oral administration comprise inert diluents, assimilable edible carriers or combinations thereof The oral composition may be prepared as a syrup or elixir. A syrup or elixir, and may comprise, for example, at least one active agent, a sweetening agent, a preservative, a flavoring agent, a dye, a preservative, or combinations thereof.

In certain preferred embodiments an oral composition may comprise one or more binders, excipients, disintegration agents, lubricants, flavoring agents, and combinations thereof. In certain embodiments, a composition may comprise one or more of the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, etc.; or combinations thereof the foregoing. When the dosage unit form is a capsule, it may contain, in addition to materials of the above type, carriers such as a liquid carrier. Various other materials may be present as coatings or to modify the physical form of the dosage unit. For instance, tablets, pills, or capsules may be coated with shellac, sugar or both.

Sterile injectable solutions can be prepared by incorporating active compounds in the required amount in the appropriate solvent optionally with various of the other ingredients enumerated above, as called for, followed by filtered sterilization. Generally, dispersions are prepared by incorporating the various sterilized active ingredients into a sterile vehicle that contains the basic dispersion medium and/or the other ingredients. In the case of sterile powders for the preparation of sterile injectable solutions, suspensions or emulsion, the preferred methods of preparation are vacuum-drying or freeze-drying techniques that yield a powder of the active ingredient plus any additional desired ingredient from a previously sterile-filtered liquid medium thereof The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

Preferably, a composition of the disclosure is stable under standard conditions of manufacture and storage, and preserved against the contaminating action of microorganisms, such as bacteria and fungi. It will be appreciated that endotoxin contamination should be kept minimally at a safe level, for example, less that 0.5 ng/mg protein.

In particular embodiments, prolonged absorption of an injectable composition can be brought about by compositions of the disclosure that comprise agents that delay absorption, such as, for example, aluminum monostearate, gelatin or combinations thereof.

### Peripheral nerves: Sciatic nerve

With respect to the sciatic nerve, the compositions of the invention increase muscle mass, improve muscle strength, and reduce muscular atrophy. In particular, the administration of GGF2 increases myofiber size and reduces fibrosis in a tissue surrounding the peripheral nerve. For example, the administration of GGF2 increases myofiber size and reduces fibrosis in skeletal muscle. Disclosed herein are compositions and methods for preventing demylination and/or inducing remyelination of damaged peripheral nerves. In certain embodiments of the compositions and methods disclosed herein, the compositions and methods are used for preventing demylination and/or inducing remyelination of a damaged sciatic nerve, including a damaged sciatic nerve in a muscle. An exemplary muscle of the disclosed methods includes, but is not limited to, skeletal muscle.

In an embodiment, the disclosure provides compositions comprising an effective amount of a neuregulin to improve muscle function in a subject at risk of incurring a peripheral nerve injury or having an existing peripheral nerve injury. Improvements of muscle function include, by way of example, and not limitation, skeletal muscle function. Examples of improved muscle function include, but are not limited to, increase in muscle mass (e.g., increase in myofiber size), muscle strength, reduction in muscle atrophy and/or a reduction in muscle fibrosis. In a preferred aspect, the neuregulin is GGF2.

### Peripheral nerves: Cavernous nerve

The rat erectile dysfunction model is a standard, accepted and well-publicized model of peripheral nerve injury. In this specific approach, the cavernous nerve is injured by forceps compression. The same compression or crush injury can be used as a model in any other peripheral nerve. In the cavernous nerve injury model the functional deficit is in erectile function. In view of the common and consistent pathophysiology of traumatic nerve injury, such cavernous nerve injury is an excellent model for prostatectomy-induced injury, as well as a general model for all traumatic peripheral nerve injuries.

Injuries to peripheral nerves induce changes within the cell bodies of sensory neurons located in the dorsal root ganglion (DRG); these changes promote survival and axonal regeneration. Under favorable conditions, for instance following a crush injury, most nerve fibers successfully regenerate. However, in many clinically relevant circumstances, traumatic or disease-induced nerve injury has a poor outcome with only a limited return of function and often with considerable delay. In such cases, neuropathic or chronic pain states can develop.

Pain is normally associated with sensory nerve injury or damage and results in guarding and immobilization of the affected area. Nociception (the neuronal signaling underlying the sensation of pain) therefore is concomitant to mechanisms for and the promotion of rapid healing, albeit triggering an unpleasant sensory and emotional experience. However, in many pathological situations, nociceptive inputs can result in functional changes that are actively detrimental to the organism.

Nerve injury results in the alteration of many of the properties of primary afferent neurons and their central connections in the spinal cord, leading to allodynia (the perception of pain from a normally innocuous stimulus), hyperalgesia (an exaggerated response to any given pain stimulus) and an expansion of the receptive field (i.e. the area that is "painful" when a stimulus is applied). The majority of chronic pain conditions arise as a result of damage to either central or peripheral nervous tissue.

Impotence, or also referred to as erectile dysfunction (ED), is a common problem affecting 20 million men in the United States alone. Penile erection is a neurovascular phenomenon dependent upon both neural integrity and functional blood vessels. Upon sexual stimulation, neurotransmitters (especially nitric oxide) are released from the cavernous nerve terminals and endothelial cells. Resultant relaxation of arterial and arteriolar smooth muscles increases arterial flow. Blood trapped within the corpora cavernosa brings the penis to an erect state.

Injury to the cavernous nerve from radical pelvic surgeries, such as for prostate, bladder or rectal cancer, is one of the most common causes of iatrogenic ED in this country. ED is a major source of morbidity after radical prostatectomy. For example, despite the introduction of nerve-sparing surgical techniques, postoperative potency rates range between 30% and 80% for men who have undergone bilateral cavernous nerve-sparing procedures for organ-confined prostate cancer (Wang, J Sex Med, 4:1085-97, 2007).

Various neuromodulatory strategies have been investigated to date; however, there are no treatments available for either neuroprotection of the cavernous nerves prior to or at the time of injury, or treatments after injury to elicit nerve regeneration (Michl et al., J Urol 176:227-31, 2006; Burnett and Lue, J Urol 176:882-7, 2006). Despite contemporary nerve-sparing modifications to surgical and radiation therapies for pelvic malignancies there is a need for new means to preserve and restore erectile function after treatment.

Well-defined pattern of cellular changes distal to the site of damage are seen, progressing from axonal and myelin sheath degeneration, macrophage invasion, phagocytoses, and Schwann cell dedifferentiation to formation of bands of Bungner. These changes modify the injured nerve's environment and its potential for regeneration of axons. Neuronal survival is facilitated by trophic factors when axons switch from a 'transmitting' mode to growth mode, expressing proteins (GAP-43, tubulin, actin), novel neuropeptides, and cytokines New strategies enhancing growth potential are required as distal nerve stump support and neuronal capacity to regenerate are not indefinite (Fu and Gordon, Mol Neurobiol. 14: 67-116, 1997).

### EXAMPLES

### Example 1: Use of GGF2 to treat muscle atrophy following peripheral nerve injury

The overall design of the study described herein is illustrated in Table 1 below.

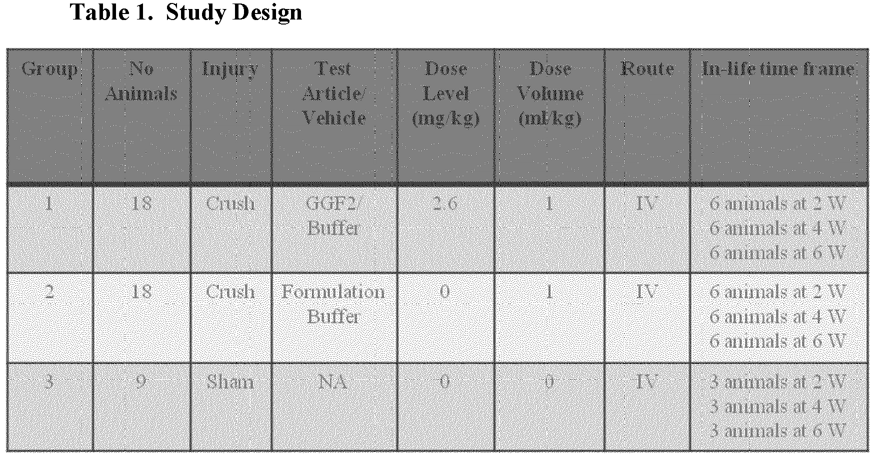

For this study, 8-10 week old male Spraque Dawley (CD) rats were used. Animals were randomized into various surgical groups based on their body weights and subsequently subjected to sciatic nerve crush injury 8-10 mm proximal to the sciatic nerve trifurcation (Figure 1) or received sham surgery on the right hind limb. Animals with sciatic nerve crush injury were treated with a single bolus dose of GGF2 (2.6 mg/kg, intravenously) or vehicle twenty four hours after injury and then twice weekly for the duration of the study. Cohorts of animals representing all treatment groups were euthanized at 2, 4 or 6 weeks following injury. Animals were deeply anesthetized with ketamine/xylazine, and euthanized by transcardial perfusion of phosphate buffered saline followed by 4% paraformaldehyde perfusion. The lateral gastrocnemius muscle and the distal sciatic nerve were collected, embedded and sectioned on the injured side. Lateral gastrocnemius muscle sections (3-5 µm) were stained with hematoxylin and eosin (H&E), Masson's trichrome, and toluidine blue for morphometric evaluation of muscle fibrosis and myofiber cross sectional area distribution (Figure 2). A significant increase in fibrosis was observed following crush injury compared to sham animals. A significant reduction in fibrosis was seen in GGF2-treated animals compared to vehicle treated animals at 4 and 6 weeks following injury. At 6 weeks, GGF2 reduced injury-induced fibrosis to near normal levels compared to vehicle treatment.

Further, the distal sciatic nerves were embedded in plastic and sectioned. Separate sections were stained with H&E, luxol fast, and toluidine blue to evaluate the extent of demyelination following crush injury. Specifically, Figure 3 shows a series of photomicrographs (Trichrome 100X) of representative muscle sections from sham [A], vehicle-treated [B], and GGF2-treated animals [C] at 2 and 6 weeks following injury. In the sham animals, the myofibers were within normal limits at 2 and 6 weeks post injury. Following crush injury, there was an increase in fibrosis and reduction in myofiber size. GGF2 treatment significantly reduced muscle fibrosis at 4 and 6 weeks post injury. Additionally, there was some improvement in the myofiber size in GGF2-treated animals compared to vehicle.

Following nerve crush injury, target muscle fibrosis increased over time. Further, myofiber size distribution within the target muscle skews toward smaller cross sectional areas in crush-injured animals compared to sham controls. GGF2 treatment significantly reduced muscle fibrosis at 4 and 6 weeks after injury.

In addition to its effects on fibrosis, GGF2 treatment promoted a shift in the myofiber area toward increased size (*i.e.* more like sham controls) at 4 and 6 weeks after injury (Figures 4A-4C). Specifically, Figure 4 shows the distribution of myofiber areas in lateral gastrocnemius muscle at 2, 4, and 6 weeks following injury. At 2 weeks post injury, the distribution of both crush-injured groups (median= 1501-2000 µm²) were distinctly different from the sham group (median= 3001-3500 µm²). At 4 and 6 weeks, the sham group showed a rightward shift until the sections exceeded 5000 µm² in area. The median of the vehicle-treated group remained shifted to smaller areas (2001-2500 µm²). GGF2 treatment significantly improved myofiber size at 4 weeks (2501-3000 µm²) and 6 weeks (3001-3500 µm²), respectively.

### Example 2: GGF2 improves remyelination of the sciatic nerve following injury

Preliminary analysis of injured distal nerve stumps showed that crush injury produced axonal demyelination at 2, 4 and 6 weeks post-injury. In addition, axonal swelling, Wallerian degeneration, mild inflammation, and fibrosis between the axons occurred following injury. Axonal damage was noted in greater than half of the nerve bundle in cross section. GGF2 improves remyelination at 4 and 6 weeks in the injured distal sciatic nerve compared to vehicle-treated controls.

Specifically, Figure 5 shows a semi-quantitative assessment of myelination in the sciatic nerve proteolipid protein stain (PLP). At 2, 4, and 6 weeks, the sciatic nerve cross sections were within normal limits in the sham animals. There were a wide variety of lesions with varying severity in the vehicle and GGF2- treated groups; however, it appeared that GGF2 qualitatively reduced the degree of demyelination when compared to vehicle-treated animals.

### Example 3: The Rat Model of Cavernous Nerve Injury

The rat model of cavernous nerve injury typically uses the following methodology. Rats are anesthetized with isoflurane. The animals are placed on a heating pad to maintain the body temperature at 37° C. The abdomen is shaved and scrubbed with antiseptic Clinidin solution (povidone iodine). A midline lower abdominal opening of peritoneal cavity is made, exposing both cavernous nerves and major pelvic ganglion (MPGs). Cavernous nerve injury is induced by crushing the cavernous nerve with a hemostat for two minutes per side. In the studies related to neuregulin, two neuregulin groups were treated 48 hours prior to injury.

The rat crush model provides a simple, reproducible and extremely reliable decrease of erectile function. This technique is used extensively and several studies have been published using this technique. There is no need to test erectile function after crush injury, decreased erectile function is predictable, and typically, functional testing is performed at about 5 weeks post crush-injury.

After injury to the cavernous nerve the abdominal cavity is closed in two layers with reapproximation of the abdominal muscles and fascia (absorbable suture) via 2-3 interrupted sutures. The skin is closed using a subcuticular (buried) running suture for the skin with a non-wicking (PDS or coated vicryl) suture material. Buprenorphine analgesic was given preemptively (10 minutes prior to procedure ending) and every 6-12 hours postoperatively for 48 hours for pain control.

At about 5 weeks postoperative, rats were anesthetized with Ketamine (100 mg/kg IP) and Xylazine (5 mg/kg). Cavernosal crura are exposed through the same incision and functional studies performed using a 23 G needle inserted into the left crura and connected to a software program specifically designed to measure intracavernous pressures. Prior to measurement, the cavernous nerves are stimulated with an electrode at 1.5 mA. Length of measurement procedure is approximately 15 minutes. The rats were euthanized with euthanyl-intercardiac before anesthetic recovery and tissues (cavernous nerves, MPG, penis, prostate) collected for light microscopy and molecular and histological assessments.

As presented in the intracavernous pressures (ICP) data shown in Fig. 6, electrostimulation of the cavernous nerves 5 weeks post-injury demonstrated significant preservation of nerve and end-organ function across both neuregulin-treated groups and this was even more significant at higher doses. The data were first analyzed by non-repeated measures ANOVA with the Bonferroni t-test and significance considered at p<0.05. All results are expressed as the mean ± SEM. Changes were also significantly improved when normalized to Aortic Pressures as shown in Fig. 7.

From a histological standpoint, data indicate that NRG treatment increased the number of intact nerve fibers based on the fluoro -gold retrogradely transported labeling in the MPG, and improved preservation of neuronal nitric oxide synthase and VaChT from nerve and smooth muscle tissues of the penis. This indicates that there are neuroprotective and/or neuroregenerative mechanism of action. Smooth muscle apoptosis is also decreased compared to crush injury animals that do not receive any neuregulin.

### Example 4: Fluoro-Gold Histology Methods

To perform this protocol, intracorporal injection of 4% fluoro-gold was performed, and at one week, Major Pelvic Ganglia (MPG) tissues were harvested and fixed in 4% paraformaldehyde, 0.1 M phosphate buffer, fixed overnight and then placed in 20% sucrose. Cryo sectioning was at 20 pm thickness. Images were taken using an Infinity camera and imaging system, followed by blinded analyses for fluoro-gold enhanced cell counts. Thereafter, MPG specimen slides were randomly selected (10 per animal) and cell counts performed to determine the number of intact neurons. (See, e.g., Dail, W. G., Trujillo, D., de la Rosa, D. and Walton, G.: Autonomic innervation of reproductive organs: analysis of the neurons whose axons project in the main penile nerve in the pelvic plexus of the rat. Anat Rec, 224: 94, 1989; Laurikainen A, Hiltunen J 0, Vanhatalo S, Klinge E, Saarma M: Glial cell line-derived neurotrophic factor is expressed in penis of adult rat and retrogradely transported in penile parasympathetic and sensory nerves. (Cell Tissue Res 2000, 302:321-9.) [0081] Thus, this was a retrograde tracing protocol using fluoro-gold. Results from this protocol provided information indicating that neuregulin treatment aided regeneration and re-projection to its target (the corpora cavernosa of the penis) and/or neuroprotection of the cavernous nerves.

Accordingly, fluoro-gold was injected into a target organ, in this case the corpora of the penis. Thereafter, uptake from the end-organ nerve terminals occurred. This uptake indicated that nerve fibers were preserved and/or re-grew into the injected area. Once there is fluoro-gold uptake, the fluorogold is transported in a retrograde fashion in the nerve axon and the label accumulated in the original neurons of the MPG (major pelvic ganglion).

Fig. 8 shows representative flour-gold labeling of major pelvic ganglia (MPG) from 3 animals per treatment group ((panel A) normal, (panel B) crush, (panel C) crush+ GGF2). Normal animals (panel A) demonstrate the amount of retrograde labeling observed in the absence of nerve injury. Crush animals (panel B) demonstrate the dramatic reduction in intact nerve fibers from the injury, as the fluoro -gold label is not able to be transported all the way back to the MPG. Crush+GGF2 animals (panel C) show an increased number of fluoro-gold labeled MPG cells, indicating that there are more preserved nerve fibers present after injury as a result of GGF2 treatment.

Fig. 9 provides a quantitation of fluoro-gold labeling in the MPG. Normal animals have a large number of cell bodies labeling in the MPG. Following a crush injury the number of labeled cells is dramatically reduced, consequent to nerve fiber damage and the resulting inability to transport retrogradely the label back to the MPG. GGF2 treatment improved the number of intact nerve fibers available to transport the fluoro-gold from the penile tissue to the MPG in a retrograde manner, resulting in a larger number of labeled cells.

### Example 5: Immunohistochemistry

Longitudinal cryosections of the proximal portion of the corpora were stained for nNos, VaChT. All washes were done with Tris buffer containing 1% triton-X. Tissue were blocked 1 hr with 5% normal goat serum then incubated overnight at 4°C with, respectively:
a) nNOs (Sigma; 1/1000) or
b) VaChT (Abcam; 1/150) or
c) TH (Millipore; 1/5000).

After several rinses, sections were incubated for 1 hr in goat-anti-rabbit HRP and donkey anti-goat (1/1000) then into a DAB solution containing 0.2% ammonium nickel sulfate and 0.03% hydrogen peroxide for 10 min. After the last wash, the sections were dehydrated, cleared in xylene and coverslipped in Permount (Fisher Scientific).

### nNos Staining:

Nitric oxide (NO) released from the axonal endplates of the cavernous nerves within the corpora cavernosa, along with endothelial NO, causes relaxation of the smooth muscle, initiating the hemodynamic changes of penile erection as well as contributing to maintained tumescence. It is currently understood that a return to potency following injury to the cavernosal nerves is dependent, at least in part, upon axonal regeneration in the remaining neural tissues and successful functional reinnervation of the end-organ (allowing neuronal NO activation). Well-defined pathobiological changes are observed in animal model studies of the penis following cavernosal nerve compromise. These patobiological changes may range from neuropraxia to lethal axonal damage, and can include apoptosis of the smooth muscle, apoptosis of the endothelium, reduced nitric oxide synthase (NOS) nerve density, up-regulation of fibroproliferative cytokines such as transforming growth factor-beta (TGF-(3), smooth muscle fibrosis or loss, or pathobiological signaling responses such as altered sonic hedgehog protein.

Additionally, a chronic absence of erection secondary to cavernosal nerve neuropraxia during the prolonged recovery phase is thought to exacerbate the potential for further cavernosal smooth muscle structural deterioration due to a failure of normal cavernosal cycling between flaccid and erect states (Bella A J, Lin G, Fandel T M, Hickling D R, Morash C, Lue T F. Nerve growth factor modulation of the cavernous nerve response to injury. J Sex Med 6 Suppl 3: 347-352, 2009).

Cavernous nNOS is a well-established marker of cavernous nerve preservation. (See, *e.g*., onlinelibrary.wiley.com/doi/10.1111/j .1464-410X.2010.09364.x/full). The results of this protocol indicated a neuroprotective and/or nerve regenerative effect following bilateral cavernous nerve injury in the rat produced according to the protocol of Example 3.

Density of staining results (representative proximal corporal sections, 5 randomly selected slides, observer blinded based on 5 animals per group) indicated preservation of nNOS staining for subjects treated with neuregulin.

Fig. 10 provides representative staining for nNos levels. Density of staining indicates presence of nNOS. Results of this work include normal tissue staining (panel A). By comparison, there is a significant loss of nNOS staining after cavernous nerve crush injury (panel B). Preserved nNOS staining of cavernous nerve endings in the penile corpora demonstrates increased rates of survival and/or regeneration of cavernous nerves following crush injury with GGF2 treatment (panel C). Density of staining indicates preservation of nNOS staining with GGF2 treatment.

### Vesicular Acetylcholine Transporter (VaChT) Staining:

Pelvic ganglion neurons that innervate the penis express nNOS and cholinergic markers, whereas sympathetic noradrenergic innervation of the penis arises largely via the sympathetic chain and does not traverse the penile nerves or pelvic ganglion. Results from this protocol provided information indicating that neuregulin treatment aided regeneration and re-projection to its target (the corpora cavernosa of the penis) and/or neuroprotection of the cavernous nerves based on intracorporal staining for vesicular acetylcholine transporter (VaChT). Although the primary etiology of postsurgical ED is neurogenic, studies in rodents have revealed that morphologic and functional changes also occur within cavernous tissue after penile nerve injury. (See, e.g., Keast J R. Plasticity of pelvic autonomic ganglia and urogenital innervation. Int Rev Cytol 2006; 248: 141-208; Andersson K E, Hedlund P, Aim P. Sympathetic pathways and adrenergic innervation of the penis. Int J Impot Res 2000; 12:55-12; Mulhall JM, Bella A J, Briganti A, McCullough A, Brock G. Erectile Function Rehabilitation in the Radical Prostatectomy Patient. J Sex Med 7(4), 1687-1698, 2010).

Density of staining results (representative proximal corporal sections, 5 randomly selected slides, observer blinded based on 5 animals per group) indicated preservation of VaChT staining in the rats who received the GGF2.

Fig. 12 provides representative immunohistochemical staining of vesicular acetylcholine transporter (VaChT). Density of staining indicates presence of VaChT. Results include normal tissue staining (panel A), and a significant loss of VaChT staining after cavernous nerve crush injury (panel B). In contrast, the preserved VaChT staining of cavernous nerve endings in the penile corpora shown in Panel C demonstrated increased rates of survival and/or regeneration of cavernous nerves following crush injury treated with GGF2 treatment (panel C). Density of staining shows indicates preservation of VaChT staining with GGF2 treatment.

### TH Staining:

TH is a marker of adrenergic nerve fibers and is used to support nerve preservation in the corpora. The proximal portion of the corpora was cryosectioned longitudinally and stained with primary antibodies raised against the catecholamine synthesis marker, tyrosine hydroxylase (Impaired Cavernous Reinnervation after Penile Nerve Injury in Rats with Features of the Metabolic Syndrome Matthew R Nangle, BSc, PhD, Joseph Proietto, MBBS, PhD, and Janet R. Keast, BSc, PhD J Sex Med 2009; 6:3032-3044).

Density of staining results indicates presence of TH. The density of staining results actually achieved (representative proximal corporal sections, 5 randomly selected slides, observer blinded based on 5 animals per group) indicated preservation of TH staining in animals treated with GGF2. Fig. 11 provides representative staining of tyrosine hydroxylase (TH) levels. The results include normal tissue staining (panel A) and, a significant loss of TH staining after cavernous nerve crush injury (panel B). Panel C shows preserved TH staining of cavernous nerve endings in the penile corpora best corresponds to a general increase in preservation of penile innervation following crush injury with GGF2 treatment (panel C). Density of staining shows trends towards preservation of TH staining with GGF2 treatment.

### Example 6: GGF2 is neuroprotective in a rat model of cavernous nerve injury-induced erectile dysfunction.

Erectile dysfunction (ED) is a common complication following radical prostatectomy. It is acknowledged to be due to penile neuropathy; however, there is no effective treatment. Glial growth factor 2 (GGF2) is a member of the neuregulin family of growth factors that has been demonstrated to protect neurons from injury and stimulate their growth in a range of animal models of neuropathy. Our previous data suggests that GGF2 delivered subcutaneously (sc) may be a viable therapy for cavernous nerve (CN) injury. In this study, an effective dose range of GGF2 was analyzed to recover erectile function (EF) and examine its effect on CN survival.

Adult male rats (on average, weighing approximately 325-350 grams) underwent bilateral cavernous nerve (CN) crush injury (BCI) or sham surgery (Control) and were divided into the following groups (n=10-12/group, blinded drug treatment): Control+vehicle (Group A); Control+GGF2 (15 mg/kg) (Group B); BCI + vehicle (Group E); BCI + GGF2 (5 mg/kg) (Group C); BCI + GGF2 (15 mg/kg) (Group D). GGF2 was administered subcutaneously (sc) 24 hours before surgery (1^{st} dose), 24 hours after surgery (2^{nd} dose) and once weekly until study end 5 weeks after BCI or sham surgery (3^{rd} through 7^{th} doses). At the end of the treatment, erectile function (EF) was examined by monitoring intracavernous pressure (ICP) responses to electrical stimulation of CN at 0.3, 1 and 4 volts. CNs were processed for electron microscopic analysis and quantitation of unmyelinated nerve fibers.

Figure 13 demonstrates the increased body weights of each experimental group from the first through the final dose of GGF2 or vehicle.

EF was significantly decreased in BCI+vehicle and BCI+GGF2 (15mg/kg) groups (p<0.05) but not in BCI+GGF2 (5mg/kg) group (p>0.05) compared with Control+vehicle at 0.3 and 1 volt (Figure 14A and B). At 4 volts, EF was significantly increased in both BCI+GGF2 (5 and 15 mg/kg) groups compared to BCI+vehicle (p>0.05) and did not differ from Control+vehicle (p>0.05) (Figure 14A and B). In BCI+GGF2 (5 mg/kg) group, the number of denervated Schwann cells without unmyelinated fibers was significantly lower than BCI+vehicle group (p<0.05), and unmyelinated nerve fiber histogram demonstrated a rightward shift, indicating an increase in number of unmyelinated axons per Schwann cell (Figure 15).

Treatment with GGF2 at 5mg/kg, sc effectively preserved EF in rats following CN crush injury with an increase in the number of surviving unmyelinated nerve fibers. These results indicate that GGF2 is a potent neuroprotective agent in penile innervation and provides a new therapeutic approach to treat or prevent ED following prostate surgery.

### Example 7: Alternative Embodiments

Peripheral nerve injury can occur in almost any surgical context. The likelihood of nerve injury is correlated with the location and extent of tissue dissection in any surgery. For example, mastectomy surgery has frequent complications resulting from peripheral nerve injury including numbness of the axilla and arm (e.g., injury to intercostobrachial nerve injury), winged scapula (injury to long thoracic nerve injury), palsy of the latissimus dorsi (injury to thoracodorsal nerve injury). (See Watt-Boolsen et al., 1988; Aitken and Minton, 1983).

Accordingly, neuregulin is used either prior to, after or both before and after mastectomy to limit injury to nerves and/or enhance recovery of peripheral nerve function. Patients scheduled to undergo mastectomy are treated about 24 hours prior to surgery with an appropriate amount of neuregulin. Optionally, patients are also treated for a period of up to about 6 weeks or more following surgery to enhance neural recovery. In alternative embodiments, patients are only treated before or only treated after surgery. As noted herein, neuregulin is used to prevent nerve injury consequent to tumor resection surgeries (prostatectomy, mastectomy, thyroidectomy, etc). It is noted that neuregulins have been implicated as promoters and as suppressors of tumor cell formation and growth (Atlas et al., 2003; Chua et al., 2009). Neuregulin treatment may or may not be contraindicated in patients with certain tumors. Neuregulins are used in patients with erbB positive tumors only when sufficient safety studies demonstrate that neuregulins do not enhance growth of such tumor.

Moreover, treatment of nerve injury from surgery is not limited to mastectomy and prostatectomy. Nerve injury frequently occurs in any surgery involving significant dissection and/or resection. These surgeries may include but are not limited to upper limb surgery, hand surgery, knee surgery/ replacement, hip surgery/replacement, elbow surgery/replacement, neck dissection for arterial and venous surgery, thyroid surgery, tonsillectomy, hand and foot surgery. Peripheral nerve injury is common with pelvic, abdominal surgery and colorectal surgery. Nerve injury also occurs with oral and facial surgeries.

In addition to direct injury to nerves through dissection and resection in surgery, nerve injury frequently results from compression or stretching of nerves during surgery due to positioning of the patient, compression on contact points or from drapes, restraints, clips, tape or any other object that may compress tissue. These may be inevitable results of the surgery or the result of improper technique. No matter what the setting or etiology of peripheral nerve injury, neuregulins are found to prevent and/or treat such injury.

In humans, clinical trials demonstrate efficacy of NRG for the prevention and treatment of peripheral nerve injury with data from assessing sensory and/or motor function of frequently affected nerve regions in patients that are treated with neuregulin or with a placebo control. For example, numbness of the axilla may be tested by standard neurological methods of sensory function including tests of allodynia, hyperalgesia, sensory threshold or acuity (two-point discrimination). These methods are standard in the field. Patients are followed for a period of several months after surgery and statistical comparisons made between groups of patients treated with neuregulin and those treated with a control. Pursuant to these trails, NRG treatment before and/or after a surgical event are found to prevent and/or treat peripheral nerve injury evaluated.

Trials analogous to the foregoing also assess in a similar fashion motor strength, range of motion and coordination. Pursuant to these trials, NRG treatment before and/or after a surgical event are found to prevent and/or treat peripheral nerve injury that results in impairment in one or more of motor strength, range of motion or coordination.

### Example 8: Use of GGF2 to decrease neuropathic pain and/or improve sensory function following peripheral nerve injury

The overall design of the study described herein is illustrated in Table 2 below.

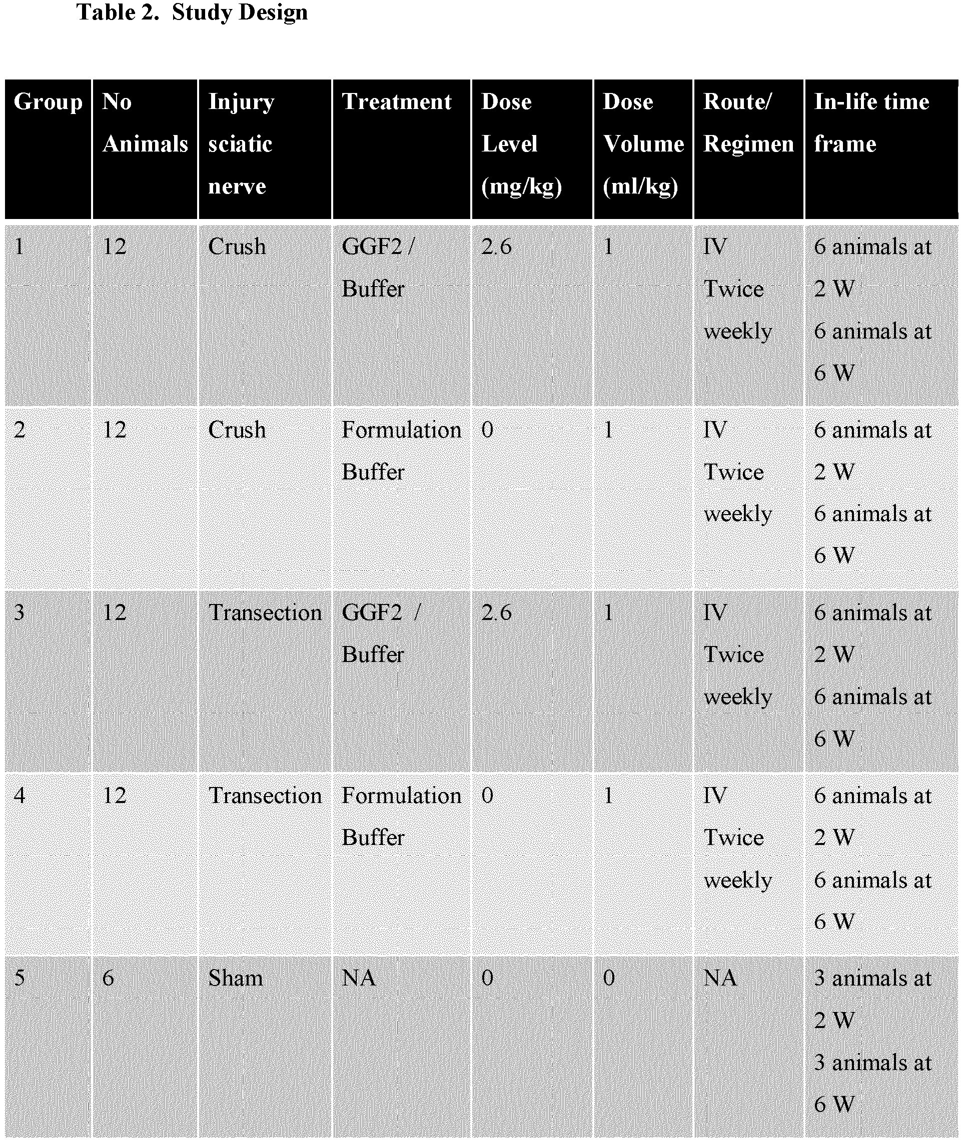

For this study, 8-10 week old male Spraque Dawley (CD) rats were used. Animals were randomized into surgical groups based on their body weights and subsequently subjected to either left sciatic nerve injury (crush or transection) 8-10 mm proximal to the sciatic nerve trifurcation (Figure 1) or received sham surgery on the left hind limb. Animals with sciatic nerve injury were treated with a single bolus dose of GGF2 (2.6 mg/kg, intravenously) or vehicle twenty four hours after injury and then twice weekly for the duration of the study. Cohorts of animals representing all treatment groups were assessed for evoked behavioral responses at baseline immediately prior to surgery and then biweekly thereafter until they were euthanized at 2 or 6 weeks following injury. Mechanical sensitivity of the ipsilateral (nerve injured) paw was assessed using von Frey filaments wherein a series of graded tactile stimuli were applied to the bottom of the paw using filaments calibrated to bend at specified forces. Tactile (mechanical) response threshold was measured by recording the force that causes the affected paw to be withdrawn abruptly. Post-injury mechanical thresholds obtained at 2, 4 and 6 weeks were averaged by group and compared across treatments.

Cold sensitivity of the ipsilateral paw was also assessed prior to and biweekly following nerve injury or sham surgery. For selective cooling of a single hind limb rats were placed individually in chambers on an elevated perforated floor surface and acetone (100 µL) was applied to hind paw. Subjects sensitive to this evaporative cooling exhibited a rapid lifting/licking of the hind paw. The percentage of positive responses evoked by five sequential applications of acetone, separated from one another by at least 10 minutes, was taken as a measure of cold sensitivity. Post-injury cold sensitivities obtained at 2, 4 and 6 weeks were averaged by group and compared across treatments.

Rats subjected to a partial sciatic nerve injury, such as sciatic crush, often develop symptoms of neuropathic pain. This was exemplified in the current study by the dramatic reduction in mechanical thresholds seen in the crush-lesioned rats (Fig 16A). Note that rats undergoing sham surgery maintained a 15 g or greater mechanical threshold while vehicle-treated crush-injured rats exhibited mechanical sensitivities considered to be in the allodynic range. Treatment of crush-injured rats with GGF2 was seen to slightly mitigate the severity of mechanical allodynia. A similar pattern was seen in the assessment of cold sensitivity following crush injury wherein GGF2-treated rats exhibited a reduced percent response to acetone evaporative cooling as compared with vehicle-treated rats (Fig 17A).

Rats subjected to complete injury of the sciatic nerve, as in transection, exhibit deafferentation symptoms such as a complete lack of mechanical and cold sensitivity. In addition, the lack of afferent sensation coupled with apparent dysesthesias occasionally engenders self-mutilation behaviors (autotomy or autophagy) directed toward the denervated extremity. In sciatic transection rats, GGF2 treatment, in contrast to vehicle treatment, was seen to readily promote the re-establishment of mechanical sensation, without producing allodynia (Fig 16B). Paw cold sensitivities were similarly enhanced by GGF2 treatment following sciatic transection (Fig 17B) in comparison with vehicle treatment. Finally, fewer rats exhibited transection-induced deafferentation autotomy when followed over 6 weeks of GGF2 treatment (1 of 6 rats) as compared to 6 weeks of vehicle treatment (2 of 6 rats).

## Claims

1. A composition comprising an effective amount of a GGF2 for use in a method to decrease neuropathic pain in a subject at risk of incurring a peripheral nerve injury due to a surgical procedure.

2. A composition for use according to claim 1 wherein the peripheral nerve is a sciatic nerve or a cavernous nerve, or wherein the peripheral nerve injury results in erectile dysfunction.

3. A composition for use according to claim 1 wherein the surgical procedure is a tissue dissection or a tumor resection, optionally wherein the tissue or tumor is a cancer, preferably wherein the cancer is a solid cancer or wherein the cancer is a prostate or a breast cancer.

4. A composition for use according to claim 1 wherein the surgical procedure is a pelvic, abdominal, or colorectal surgery.

5. A composition for use according to claim 1 wherein the peripheral nerve injury is a nerve transection, a nerve crush, or a nerve demyelination.

6. A composition for use according to claim 1, wherein in the method the composition is administered according to a discontinuous dosing regimen.

7. A composition for use according to claim 6 wherein the discontinuous dosing regimen comprises administering the composition at specified intervals, optionally wherein
a) the specified intervals are selected from the group consisting of every 24 hours, every 48 hours, and every 72 hours, or the group consisting of at least every 24 hours, at least every 48 hours, and at least every 72 hours, and preferably wherein the discontinuous dosing regimen continues for a period of time selected from the group consisting of about 1 week, about 2 weeks, about 3 weeks, about 4 weeks, about 5 weeks, about 6 weeks, about 7 weeks, about 8 weeks, about 9 weeks, about 10 weeks, about 11 weeks, and about 12 weeks, or
b) the specified interval is at least once per week, and preferably wherein the discontinuous dosing regimen continues for a period of time selected from the group consisting of about 1 month, about 2 months, or about 3 months.

8. A composition for use according to claim 1 wherein the effective amount of a GGF2 is between about 0.001 mg/kg of body weight to about 5 mg/kg of body weight.

9. A composition for use according to claim 8, wherein the effective amount of a GGF2 is between about 0.001 mg/kg of body weight to about 0.5 mg/kg of body weight.

10. A composition for use according to claim 8, wherein the effective amount of a GGF2 is selected from
a) from about 0.06 mg/kg to about 0.1 mg/kg,
b) from about 0.1 mg/kg to about 0.3 mg/kg,
c) from about 0.3 mg/kg to about 0.5 mg/kg,
d) from about 0.5 mg/kg to about 0.7 mg/kg,
e) from about 0.7 mg/kg to about 1.0 mg/kg,
f) from about 0.5 mg/kg to about 1.0 mg/kg, or
g) from about 1.0 mg/kg to about 1.5 mg/kg.

## Patentansprüche

1. Zusammensetzung umfassend eine wirksame Menge eines GGF2 zur Verwendung in einem Verfahren zur Verminderung neuropathischer Schmerzen bei einer Person, bei der das Risiko besteht, eine Verletzung eines peripheren Nervs aufgrund eines chirurgischen Eingriffs zu erleiden.

2. Zusammensetzung zur Verwendung nach Anspruch 1, wobei der periphere Nerv ein Ischiasnerv oder ein kavernöser Nerv ist oder wobei die Verletzung des peripheren Nervs zu einer erektilen Dysfunktion führt.

3. Zusammensetzung zur Verwendung nach Anspruch 1, wobei das chirurgische Verfahren eine Gewebedissektion oder eine Tumorresektion ist, wobei das Gewebe oder der Tumor optional ein Krebs ist, wobei der Krebs vorzugsweise ein solider Krebs ist oder wobei der Krebs ein Prostata- oder Brustkrebs ist.

4. Zusammensetzung zur Verwendung nach Anspruch 1, wobei das chirurgische Verfahren eine Becken-, Bauch- oder kolorektale Operation ist.

5. Zusammensetzung zur Verwendung nach Anspruch 1, wobei die Verletzung des peripheren Nervs eine Nervendurchtrennung, eine Nervenquetschung oder eine Nervendemyelinisierung ist.

6. Zusammensetzung zur Verwendung nach Anspruch 1, wobei bei dem Verfahren die Zusammensetzung nach einem diskontinuierlichen Dosierungsschema verabreicht wird.

7. Zusammensetzung zur Verwendung nach Anspruch 6, wobei das diskontinuierliche Dosierungsschema die Verabreichung der Zusammensetzung in festgelegten Intervallen umfasst, wobei wahlweise
a) die festgelegten Intervalle aus der Gruppe bestehend aus alle 24 Stunden, alle 48 Stunden und alle 72 Stunden oder der Gruppe bestehend aus mindestens alle 24 Stunden, mindestens alle 48 Stunden und mindestens alle 72 Stunden ausgewählt werden, und wobei das diskontinuierliche Dosierungsschema vorzugsweise über einen Zeitraum fortgesetzt wird, der aus der Gruppe bestehend aus etwa einer Woche, etwa 2 Wochen, etwa 3 Wochen, etwa 4 Wochen, etwa 5 Wochen, etwa 6 Wochen, etwa 7 Wochen, etwa 8 Wochen, etwa 9 Wochen, etwa 10 Wochen, etwa 11 Wochen und etwa 12 Wochen ausgewählt wird, oder
b) das festgelegte Intervall mindestens einmal pro Woche ist und wobei das diskontinuierliche Dosierungsschema vorzugsweise über einen Zeitraum fortgesetzt wird, der aus der Gruppe bestehend aus etwa einem Monat, etwa 2 Monaten oder etwa 3 Monaten ausgewählt wird.

8. Zusammensetzung zur Verwendung nach Anspruch 1, wobei die wirksame Menge eines GGF2 zwischen etwa 0,001 mg/kg Körpergewicht bis etwa 5 mg/kg Körpergewicht liegt.

9. Zusammensetzung zur Verwendung nach Anspruch 8, wobei die wirksame Menge eines GGF2 zwischen etwa 0,001 mg/kg Körpergewicht und etwa 0,5 mg/kg Körpergewicht liegt.

10. Zusammensetzung zur Verwendung nach Anspruch 8, wobei die wirksame Menge eines GGF2 ausgewählt wird aus
a) von etwa 0,06 mg/kg bis etwa 0,1 mg/kg,
b) von etwa 0,1 mg/kg bis etwa 0,3 mg/kg,
c) von etwa 0,3 mg/kg bis etwa 0,5 mg/kg,
d) von etwa 0,5 mg/kg bis etwa 0,7 mg/kg,
e) von etwa 0,7 mg/kg bis etwa 1,0 mg/kg,
f) von etwa 0,5 mg/kg bis etwa 1,0 mg/kg, oder
g) von etwa 1,0 mg/kg bis etwa 1,5 mg/kg.

## Revendications

1. Une composition comprenant une quantité efficace d'un GGF2 (facteur de croissance glial 2) pour utilisation dans un procédé pour diminuer la douleur neuropathique chez un sujet risquant de subir une lésion du nerf périphérique due à une intervention chirurgicale.

2. Une composition pour utilisation selon la revendication 1 dans laquelle le nerf périphérique est un nerf sciatique ou un nerf caverneux, ou dans laquelle la lésion du nerf périphérique entraîne un dysfonctionnement érectile.

3. Une composition pour utilisation selon la revendication 1 dans laquelle l'intervention chirurgicale est une dissection de tissu ou une résection de tumeur, éventuellement dans laquelle le tissu ou la tumeur est un cancer, de préférence dans laquelle le cancer est un cancer solide ou dans laquelle le cancer est un cancer de la prostate ou du sein.

4. Une composition pour utilisation selon la revendication 1 dans laquelle l'intervention chirurgicale est une chirurgie pelvienne, abdominale, ou colorectale.

5. Une composition pour utilisation selon la revendication 1 dans laquelle la lésion du nerf périphérique est une section transversale de nerf, un écrasement de nerf, ou une démyélinisation de nerf.

6. Une composition pour utilisation selon la revendication 1, dans laquelle dans le procédé, la composition est administrée selon un schéma posologique discontinu.

7. Une composition pour utilisation selon la revendication 6 dans laquelle le schéma posologique discontinu consiste à administrer la composition à des intervalles spécifiés, éventuellement dans laquelle
a) les intervalles spécifiés sont choisis dans le groupe constitué par toutes les 24 heures, toutes les 48 heures, et toutes les 72 heures, ou dans le groupe constitué par au moins toutes les 24 heures, au moins toutes les 48 heures, et au moins toutes les 72 heures, et de préférence dans laquelle le schéma posologique discontinu se poursuit pendant une période de temps choisie dans le groupe constitué par environ 1 semaine, environ 2 semaines, environ 3 semaines, environ 4 semaines, environ 5 semaines, environ 6 semaines, environ 7 semaines, environ 8 semaines, environ 9 semaines, environ 10 semaines, environ 11 semaines, et environ 12 semaines, ou
b) l'intervalle spécifié est d'au moins une fois par semaine, et de préférence dans laquelle le schéma posologique discontinu se poursuit pendant une période de temps choisie dans le groupe constitué par environ 1 mois, environ 2 mois, ou environ 3 mois.

8. Une composition pour utilisation selon la revendication 1 dans laquelle la quantité efficace d'un GGF2 est entre environ 0,001 mg/kg de poids corporel à environ 5 mg/kg de poids corporel.

9. Une composition pour utilisation selon la revendication 8, dans laquelle la quantité efficace d'un GGF2 est entre environ 0,001 mg/kg de poids corporel à environ 0,5 mg/kg de poids corporel.

10. Une composition pour utilisation selon la revendication 8, dans laquelle la quantité efficace d'un GGF2 est choisie parmi
a) d'environ 0,06 mg/kg à environ 0,1 mg/kg,
b) d'environ 0,1 mg/kg à environ 0,3 mg/kg,
c) d'environ 0,3 mg/kg à environ 0,5 mg/kg,
d) d'environ 0,5 mg/kg à environ 0,7 mg/kg,
e) d'environ 0,7 mg/kg à environ 1,0 mg/kg,
f) d'environ 0,5 mg/kg à environ 1,0 mg/kg, ou
g) d'environ 1,0 mg/kg à environ 1,5 mg/kg.


### SEQUENCE LISTING

<110> Acorda Therapeutics, Inc. Caggiano, Anthony Bella, Anthony Iaci, Jennifer Ganguly, Anindita Parry, Thomas Colburn, Ray
<120> Use of Neuregulin to Treat Peripheral Nerve Injury
<130> 43509-525001WO
<150> 61/618,381
   <151> 2012-03-30
<150> 61/674,060
   <151> 2012-07-20
<150> 61/693,589
   <151> 2012-08-27
<150> 61/693,585
   <151> 2012-08-27
<150> 61/785,419
   <151> 2013-03-14
<160> 1
<170> PatentIn version 3.5
<210> 1
   <211> 422
   <212> PRT
   <213> Homo sapiens
<400> 1
   Met Arg Trp Arg Arg Ala Pro Arg Arg Ser Gly Arg Pro Gly Pro Arg
   1               5                   10                  15
   Ala Gln Arg Pro Gly Ser Ala Ala Arg Ser Ser Pro Pro Leu Pro Leu
               20                  25                  30
   Leu Pro Leu Leu Leu Leu Leu Gly Thr Ala Ala Leu Ala Pro Gly Ala
           35                  40                  45
   Ala Ala Gly Asn Glu Ala Ala Pro Ala Gly Ala Ser Val Cys Tyr Ser
       50                  55                  60
   Ser Pro Pro Ser Val Gly Ser Val Gln Glu Leu Ala Gln Arg Ala Ala
   65                  70                  75                  80
   Val Val Ile Glu Gly Lys Val His Pro Gln Arg Arg Gln Gln Gly Ala
                   85                  90                  95
   Leu Asp Arg Lys Ala Ala Ala Ala Ala Gly Glu Ala Gly Ala Trp Gly
               100                 105                 110
   Gly Asp Arg Glu Pro Pro Ala Ala Gly Pro Arg Ala Leu Gly Pro Pro
           115                 120                 125
   Ala Glu Glu Pro Leu Leu Ala Ala Asn Gly Thr Val Pro Ser Trp Pro
       130                 135                 140
   Thr Ala Pro Val Pro Ser Ala Gly Glu Pro Gly Glu Glu Ala Pro Tyr
   145                 150                 155                 160
   Leu Val Lys Val His Gln Val Trp Ala Val Lys Ala Gly Gly Leu Lys
                   165                 170                 175
   Lys Asp Ser Leu Leu Thr Val Arg Leu Gly Thr Trp Gly His Pro Ala
               180                 185                 190
   Phe Pro Ser Cys Gly Arg Leu Lys Glu Asp Ser Arg Tyr Ile Phe Phe
           195                 200                 205
   Met Glu Pro Asp Ala Asn Ser Thr Ser Arg Ala Pro Ala Ala Phe Arg
       210                 215                 220
   Ala Ser Phe Pro Pro Leu Glu Thr Gly Arg Asn Leu Lys Lys Glu Val
   225                 230                 235                 240
   Ser Arg Val Leu Cys Lys Arg Cys Ala Leu Pro Pro Gln Leu Lys Glu
                   245                 250                 255
   Met Lys Ser Gln Glu Ser Ala Ala Gly Ser Lys Leu Val Leu Arg Cys
               260                 265                 270
   Glu Thr Ser Ser Glu Tyr Ser Ser Leu Arg Phe Lys Trp Phe Lys Asn
           275                 280                 285
   Gly Asn Glu Leu Asn Arg Lys Asn Lys Pro Gln Asn Ile Lys Ile Gln
       290                 295                 300
   Lys Lys Pro Gly Lys Ser Glu Leu Arg Ile Asn Lys Ala Ser Leu Ala
   305                 310                 315                 320
   Asp Ser Gly Glu Tyr Met Cys Lys Val Ile Ser Lys Leu Gly Asn Asp
                   325                 330                 335
   Ser Ala Ser Ala Asn Ile Thr Ile Val Glu Ser Asn Ala Thr Ser Thr
               340                 345                 350
   Ser Thr Thr Gly Thr Ser His Leu Val Lys Cys Ala Glu Lys Glu Lys
           355                 360                 365
   Thr Phe Cys Val Asn Gly Gly Glu Cys Phe Met Val Lys Asp Leu Ser
       370                 375                 380
   Asn Pro Ser Arg Tyr Leu Cys Lys Cys Pro Asn Glu Phe Thr Gly Asp
   385                 390                 395                 400
   Arg Cys Gln Asn Tyr Val Met Ala Ser Phe Tyr Ser Thr Ser Thr Pro
                   405                 410                 415
   Phe Leu Ser Leu Pro Glu
               420